# EUROPEAN PATENT APPLICATION

(11) **EP 4 477 672 A1**
(43) Date of publication of application: **18.12.2024**
(21) Application number: 22925697.9
(22) Date of filing: 25.11.2022
(51) Int. Cl.: C07K 19/00, C12N 7/01, C12N 5/10, A61K 39/00, A61P 35/00, C12R 1/93

(54) **CONSTRUCTION AND APPLICATION OF NOVEL BISPECIFIC CHIMERIC ANTIGEN RECEPTOR**

(30) Priority: 11.02.2022 CN 202210126836
(71) Applicant: Peking University Shenzhen Graduate School, Guangdong 518055 (CN)
(72) Inventor: CAO, Yu, Shenzhen, Guangdong 518055 (CN); ZHAO, Lijun, Shenzhen, Guangdong 518055 (CN); QI, Xuexiu, Shenzhen, Guangdong 518055 (CN); LI, Shuhong, Shenzhen, Guangdong 518055 (CN)
(74) Representative: Bjerkén Hynell KB
(86) International application number: PCT/CN2022/134326
(87) International publication number: WO 2023/151346

(57) **Abstract**

The present invention provides construction and application of a novel bispecific chimeric antigen receptor (CAR), and relates to the technical field of immunotherapy. The CAR provided by the present invention comprises an antigen-binding domain, a linker peptide, a hinge region, a transmembrane domain, a 4-1BB costimulatory signaling region, and a CD3ζ signaling domain. The antigen-binding domain comprises an anti-CD 19 scFv and an anti-CD22 nano antibody, or comprises an anti-HER2 scFv and a ligand capable of identifying IGF 1R. A CAR-T provided by the present invention has the capability of simultaneously binding to two target antigens, and has stronger killer activity and cytokine release capability compared with a single-target CAR-T. A bispecific CAR-T cell provided by the present invention is a bispecific CAR-T cell based on an antiparallel β-stranded loop linker (BS Loop), and the two antigen-binding domains can both play a good role, without affecting each other, and have stronger tumor killer activity and T cell activation and proliferation capacity compared with other linear tandem bispecific CAR-T cells or reported bispecific CAR-T cells of the BS Loop.

## Description

The present application claims priority based on Chinese Patent Application No. 202210126836.0, filed with the China National Intellectual Property Administration (CNIPA) on February 11, 2022. The Chinese Patent Application is titled "CONSTRUCTION METHOD AND USE OF NOVEL BISPECIFIC CHIMERIC ANTIGEN RECEPTOR (CAR)" and is hereby incorporated by reference in its entirety.

### TECHNICAL FIELD

The present disclosure pertains to the technical field of immunotherapy, specifically focusing on a construction method and use of a novel bispecific chimeric antigen receptor (CAR).

### BACKGROUND

Chimeric antigen receptor (CAR)-T cell immunotherapy, or CAR-T cell therapy for short, is a groundbreaking precision-targeted treatment for cancer. In recent years, this therapy has shown remarkable results in the clinic due to continuous optimization and refinement, making it a highly-promising novel tumor immunotherapy with the potential to provide rapid, precise, and possibly curative effects. CAR-T therapy involves the use of genetic engineering to introduce a specific antigen recognition domain along with signaling elements that activate T cells into the patient's T cells. This modification enables the T cells to directly engage and be activated by the tumor cells expressing the corresponding antigen on their surface. Once activated, these T cells release perforin and granzyme B to directly kill the tumor cells. Additionally, they secrete cytokines that attract other innate immune cells to the tumor site, further enhancing the destruction of cancer cells. Furthermore, CAR-T therapy can generate memory T cells, providing a long-lasting and specific anti-tumor response. CAR therapy was first proposed by Gross *et al.* in the late 1980s. The emergence of immune cell therapies such as LAK, TIL, and CIK laid the foundation for research on the following CAR-T therapy. So far, DC-CIK, CTL, TCR-T, CAR-NK, and CAR-macrophages are also research directions in immune cell therapy in addition to the CAR-T therapy. However, the CAR-T therapy is still a current focus of academia and industry from the perspective of technology maturity and application prospects. The CAR-T therapy exhibits a significant efficacy in the treatment of acute leukemia and non-Hodgkin's lymphoma, and has shown desirable targeting, killing, and persistence in *in vitro* and clinical trials, thus demonstrating huge application potentials and development prospects.

Acute lymphoblastic leukemia (ALL), as a malignant tumor originating from the abnormal proliferation of B-lineage or T-lineage lymphocytes in the bone marrow, is a hematoma causing lesions that involve the surrounding blood, lymph nodes, and organs systemically. Relapsed/refractory B-lineage acute lymphoblastic leukemia (B-ALL) generally shows high long-term morbidity and poor prognosis. Cluster of differentiation (CD) refers to cell surface markers that appear or disappear on leukocytes of different lineages at different stages of normal differentiation and maturation and during activation. CD molecules can be used as surface markers to identify cell types. CD19 is specifically expressed on normal B cells and B cell precursor cells, but not on hematopoietic stem cells and non-hematopoietic cells. CD19 also serves as an ideal therapeutic target, and has been proven to show a significant clinical efficacy in the ALL by targeting CAR-T cell immunotherapy and bispecific antibody drugs. Clinical studies have registered multiple trials targeting CD 19 as a CAR-T cell target. When the B-ALL are treated using CAR-T cells (Kymriah, Tisagenlecleucel, CTL019) targeting CD19 (clone: FMC63), 70% to 90% of patients achieve complete remission. However, still 30% of the patients eventually relapsed due to CD19-negative tumor cells, which may be related to the mutation of tumor cells and the expansion of CD19-negative tumor cells. Relapse caused by loss or down-regulation of target antigen is a key vulnerability of the current CAR-T technology. CD22 expression is restricted to the B cell lineage, and can be found in most B-ALL patients and even most patients with relapse after immune escape after receiving CD19-targeted immunotherapy. CD22-targeted CAR-T cells can mediate similar anti-leukemia effects and safety in patients who relapse due to CD19 down-regulation or CD19 loss after CD19-directed immunotherapy. Compared with single-targeting CAR-T cells, CAR-T cells that simultaneously target CD19 and CD22 can produce a synergistic effect and overcome the differential expression of target antigens between patients and the relapse caused by antigen escape. Based on this, the efficacy and treatment time are maximized while attacking tumor cells, as well as uneven expression and low expression of targets are avoided during single-target therapy. The structural design (for example: scFv clone selection, scFv sequence, type and length of connecting peptide (C-peptide), ligation in parallel and series, or cyclic configuration) of bispecific CAR-T cells targeting CD19 and CD22 demonstrates a significant impact on the function of CAR-T cells. Currently, CD19/CD22-targeting bispecific CAR-T cell therapy is less applied clinically. The difficulty in applications lies in constructing a CAR molecular configuration, which can simultaneously recognize and bind two tumor antigens and have two antigen-binding domains that do not affect each other. Most bispecific CAR-T cells are in a linear structure, which may attract inhibitory phosphatases to destroy the formation of synapses if immune synapses formed by the CAR-T cells are excessively wide apart. As a result, the antigen domain located at a distal end of the CAR-T cell generally cannot bind to the antigen well to form the immune synapses, thus ultimately causing a target at the distal end failed to achieve targeted therapy well.

Currently, there are few ideal targets for solid tumors. As a member of the human epidermal growth factor receptor family, Her2 is less or even not expressed in normal tissues, but highly expressed in a variety of tumors (including breast cancer, lung cancer, prostate cancer, head and neck squamous cell carcinoma, gastrointestinal tumors, and gynecological tumors), making it a potential target for the CAR-T therapy. Human epidermal growth factor receptor 2 is also known as ErbB-2, c-erbB2, or Her2/neu. *Her2* gene is located on chromosome 17q21, with a full length of 28,515 bp, transcribing 4,624 nt of mRNA, and encoding a 185 kDa protein (p185). The first humanized monoclonal antibody targeting anti-Her2, trastuzumab, has affected the diagnosis and treatment model of breast cancer, which is a major breakthrough in drug treatment of breast cancer. Moreover, in February 2013, Ado-trastuzumab emtansine, a monoclonal antibody as well as a microtubule inhibitor conjugate targeting the Her2, was approved in the United States. In December 2018, pertuzumab, a humanized monoclonal antibody targeting Her2, was approved for marketing by the Food and Drug Administration approved the marketing of pertuzumab in China, which blocks the cell cycle and induces apoptosis by inhibiting the ligand-dependent heterodimerization between Her2 and its family members. In addition, drugs that target Her2 or co-target Her2, EGFR and other receptors, such as lapatinib, pyrotinib, and neratinib, have achieved excellent results in clinical applications. However, the above approved drugs are currently only used for tumor patients with high Her2 expression level, while showing poor prognosis on patients with low Her2 expression level or antigen heterogeneity and with Her2 antigen escape caused by targeted therapy. Studies have proved that about 30% of Her2-overexpressing tumors carry a p95Her2 spliceosome, which serves as a prognostic marker for Her2-positive tumors, thereby developing resistance to treatments with Her2-targeted drugs already on the market (including trastuzumab, pertuzumab, and Ado-trastuzumab emtansine). This resistance is similar to that exhibited by Her2-negative tumor cells. In addition, Her2-targeted CAR-T cell products are currently available for the treatment of preclinical and clinical cancer patients. However, after receiving traditional CAR-T therapy, the Her2-positive cell antigen mutates, making the CAR-T cells unable to successfully recognize tumor cells, and resulting in immune evasion. Similarly, there is also reduced expression levels of Her2 on tumor surfaces in addition to the antigen modification. It is reported that Her2 receptors on the surface of tumor cells can interact with receptors including other Her family receptors, MET, and insulin-like growth factor 1 receptor (IGF1R), thereby affecting downstream signaling functions. The IGF1R receptor is abnormally expressed in various malignant tumors such as acute leukemia and breast cancer. A variety of evidence shows that the IGF1R signaling pathway is mediated by multiple mechanisms and then involved in tumorigenesis, mitosis, metastasis, angiogenesis, and anti-apoptosis, as well as tumor drug resistance, radiotherapy resistance, and Her2-targeted therapy. The IGF1R and Her2 can induce severe tumor invasion and potential resistance to Her2-targeted therapies synergistically. The combination therapeutic strategies targeting Her2 and IGF1R have emerged due to the synergy of these two tyrosine kinase receptors. Moreover, antibodies and small molecule inhibitors based on Her2/IGF1R-combined targeted therapy have exerted excellent anti-tumor effects in preclinical studies. The Her2/IGF1R dual-targeting drug demonstrates a significant anti-tumor activity in breast cancer, suggesting that drugs with a broader receptor specificity may be more effective than single-receptor-selective drugs. Although the Her2/IGF1R dual-target inhibition strategy has been elaborated on the molecular mechanism and other aspects, Her2/IGF1R synergistic targeting has not yet been extended to immunotherapy or even CAR-T therapy strategies. Considering the broad spectrum of IGF1R expression, the CAR-T cells targeting Her2/IGF1R may potentially lead to targeted toxicity *in vivo.*

### SUMMARY

In light of the foregoing, the present disclosure aims to provide a novel bispecific CAR-T cell based on the ligation design of an antiparallel β-stranded loop (BS Loop) linker. The innovative bispecific CAR-T cell is adept at simultaneously targeting CD19 and CD22 antigens or targeting Her2 while recognizing an IGF1R receptor, enabling both types of antigen-binding domains to function effectively without mutual interference. Compared to other bispecific CAR-T cells arranged in linear tandem or using reported loop ligation techniques, the bispecific CAR-T cell of the present disclosure exhibits superior tumor killing activity, as well as T cell activation and proliferation capabilities.

To attain the aforementioned objective, the present disclosure provides the following technical solutions:
The present disclosure presents a CAR comprising: an antigen-binding domain, a connecting peptide (C-peptide), a hinge region, a transmembrane domain, a 4-1BB co-stimulatory signaling domain, and a CD3ζ signaling domain; wherein the antigen-binding domain encompasses a dual-target recognition molecule.

In various embodiments, the dual-target recognition molecule encompasses a fibronectin, an affibody, a lipocalin, a bicyclic peptide, an ankyrin repeat protein, a Fyn kinase derivative, a Kunitz domain, an E7 immune protein, a lymphocyte receptor variable region, a single domain antibody, a complete antibody, an antibody fragment, and an aptamer.

In some embodiments, the antigen or receptor recognized by the dual-target recognition molecule comprises any two selections from the group encompassing CD19, CD20, CD22, CD33, BCMA, CD123, CD133, CD38, CD39, CD138, CD73, CD30, CD7, CD33, CS1, CD56, CD4, CLL1, Lewis Y, CD138, ROR1, Her2/neu, IGF1R, EGFR, Her3, Her4, VEGFR-1, VEGFR-2, VEGFR-3, mesothelin, GPC2, GPC3, CD33/IL3Ra, c-Met, MUC-1, PSMA, CAIX, CEA, PSCA, GD2, glycolipid F77, EGFRvIII, EpCAM, CD70, Claudin 18.2, IL-13, CD133, CD171, FAP, FBP, PD-L1, NYESO-1, and MAGEA3.

In certain embodiments, the C-peptide encompasses a G4S Linker, a Long Hinge Lama Linker, and a β-Stranded Loop Linker; with forward and reverse amino acid sequences of the β-Stranded Loop Linker including EETKKYQS and SYTYNYEK.

In certain embodiments, the antigen-binding domain comprises an anti-CD19 scFv and an anti-CD22 nanobody, or an anti-Her2 scFv and a ligand capable of recognizing IGF1R.

In some embodiments, the antigen-binding domain comprises the anti-CD22 nanobody and the anti-CD19 scFv sequentially ligated in series with the C-peptide, or a variable region of the light chain (VL) of the anti-CD19 scFv, the anti-CD22 nanobody, and a variable region of the heavy chain (VH) of the anti-CD19 scFv sequentially ligated in series with the C-peptide, or the VH of the anti-CD19 scFv, the anti-CD22 nanobody, and the VL of the anti-CD19 scFv sequentially ligated in series with the C-peptide.

In certain embodiments, the anti-CD19 scFv encompasses an anti-CD19 monoclonal antibody FMC63, while the anti-CD22 nanobody comprises anti-CD22 monoclonal antibodies like CD22-Nb25, CD22-Nb35, and CD22-Nb45.

In some embodiments, the antigen-binding domain comprises the ligand capable of recognizing IGF1R and the anti-Her2 scFv sequentially ligated in series with the C-peptide, or the anti-Her2 scFv and the ligand capable of recognizing IGFIR sequentially ligated in series with the C-peptide, or a VL of the anti-Her2 scFv, the ligand capable of recognizing IGF1R, and a VH of the anti-Her2 scFv sequentially ligated in series with the C-peptide, or the VH of the anti-Her2 scFv, the ligand capable of recognizing IGF1R, and the VL of the anti-Her2 scFv sequentially ligated in series with the C-peptide.

In certain embodiments, the anti-Her2 scFv comprises an anti-Her2 monoclonal antibody 4D5, while the ligand capable of recognizing IGF1R is Adnectin.

In certain embodiments, the CAR encompasses amino acid sequences as set forth in SEQ ID NO: 1 to SEQ ID NO: 5 and SEQ ID NO: 10 to SEQ ID NO: 12.

The present disclosure further provides a recombinant lentivirus, where the recombinant lentivirus is obtained by co-transfection of a viral vector with the CAR and a mammalian cell.

The present disclosure further provides a CAR-T cell, where the CAR-T cell expresses the CAR; alternatively, the genome of the CAR-T cell integrates the amino acid sequences of the CAR; or, the CAR-T cell incorporates the recombinant lentivirus.

The present disclosure further provides use of the CAR, the recombinant lentivirus, or the CAR-T cell for drug preparation.

### Beneficial effects of the present disclosure

In the present disclosure, both the CD19/CD22 CAR-T and Her2/IGF1R CAR-T cells demonstrated the capacity to bind two target antigens simultaneously, showcasing superior killing activity and cytokine release compared to single-target CAR-T cells.

In the present disclosure, both the CD19/CD22 CAR-T and Her2/IGF1R CAR-T are bispecific CAR-T cells based on the ligation design of an antiparallel β-stranded loop (BS Loop) linker. Both types of the antigen-binding domain function effectively without mutual interference. Compared to other bispecific CAR-T cells arranged in linear tandem or using reported loop ligation techniques, the bispecific CAR-T cells of the present disclosure exhibit stronger killing activity, enhanced activation, and more desirable cytokine release capabilities.

In the present disclosure, the anti-CD22 nanobody is ligated to the anti-CD19 scFv for the first time to construct the CD19/CD22 CAR-T cell, thus averting potential mismatching between two scFvs inherent in traditionally bispecific CAR-T construction with these two scFvs. Moreover, the nanobody exhibits extensive sequence identity with the human antibody heavy-chain gene family III and shows low immunogenicity. Compared to the CD19 single-target CAR-T cells like Kymriah (CTL019) already on the market, the cells of the present disclosure possess the ability to circumvent immune escape caused by loss or reduction of CD19 antigen in single-target therapy.

In the present disclosure, a ligand capable of recognizing IGF1R is ligated to the anti-Her2 scFv for the first time to construct Her2/IGF1R CAR-T cells. Due to the small molecular weight of IGF1R and absence of mismatch between the light chain and heavy chain, the cells of the present disclosure may avoid immune escape caused by the loss or decline of the Her2 antigen in single-target therapy compared to single-target Her2 CAR-T in the prior art.

### BRIEF DESCRIPTION OF THE DRAWINGS

FIG. 1 illustrates a schematic structural diagram depicting six types of CAR-T cells targeting CD19 and/or CD22;
FIG. 2 presents a schematic diagram illustrating six types of CAR molecular domains targeting CD19 and/or CD22;
FIG. 3 displays the expression and binding capabilities of six types of CAR-T cells targeting CD19 and/or CD22;
FIG. 4 demonstrates the killing activities of three types of CD19/CD22 bispecific CAR-T cells against different tumor cell lines;
FIG. 5 demonstrates the killing activities of three types of CD19/CD22 bispecific CAR-T cells on primary tumor cells from different patients;
FIG. 6 demonstrates the killing activities of CD19/CD22-targeting BS Loop CAR-T and single-target CAR-T cells against different tumor cell lines;
FIG. 7 demonstrates the killing activities of CD19/CD22-targeting BS Loop CAR-T and single-target CAR-T cells against primary tumor cells from different patients;
FIG. 8 demonstrates the killing activities of CD19/CD22-targeting BS Loop CAR-T and M971-FMC63 Loop CAR-T cells against different tumor cell lines;
FIG. 9 demonstrates the killing activities of CD19/CD22-targeting BS Loop CAR-T and M971-FMC63 Loop CAR-T cells against primary tumor cells from different patients;
FIG. 10 demonstrates the cytokine release capabilities of three types of CD19/CD22 bispecific CAR-T cells against different tumor cell lines;
FIG. 11 demonstrates the cytokine release capabilities of three types of CD19/CD22 bispecific CAR-T cells against primary tumor cells from different patients;
FIG. 12 demonstrates cytokine release capabilities of CD19/CD22-targeting BS Loop CAR-T and single-target CAR-T cells against different tumor cell lines;
FIG. 13 demonstrates the cytokine release capabilities of CD19/CD22-targeting BS Loop CAR-T and single-target CAR-T cells against primary tumor cells from different patients;
FIG. 14 demonstrates the cytokine release capabilities of CD19/CD22-targeting BS Loop CAR-T and M971-FMC63 Loop CAR-T cells against different tumor cell lines;
FIG. 15 demonstrates the cytokine release capabilities of CD19/CD22-targeting BS Loop CAR-T and M971-FMC63 Loop CAR-T cells against primary tumor cells from different patients;
FIG. 16 illustrates a comparison of the activation levels of three types of CD19/CD22 bispecific CAR-T cells;
FIG. 17 illustrates a comparison of the proliferation capabilities of three types of CD19/CD22 bispecific CAR-T cells;
FIG. 18 illustrates a comparison of the expansion capabilities of three types of CD19/CD22 bispecific CAR-T cells;
FIG. 19 presents imaging results of mice demonstrating the *in vivo* anti-tumor activity of four types of CAR-T cells targeting CD19 and/or CD22;
FIG. 20 presents fluorescence quantitative results of the *in vivo* anti-tumor activity of four types of CAR-T cells targeting CD19 and/or CD22;
FIG. 21 illustrates survival curves of mice depicting the *in vivo* anti-tumor activity of four types of CAR-T cells targeting CD19 and/or CD22;
FIG. 22 presents imaging results of mice showing the *in vivo* anti-double tumor immune escape activity of four types of CAR-T cells targeting CD19 and/or CD22;
FIG. 23 presents fluorescence quantitative results of the *in vivo* anti-double tumor immune escape activity of four types of CAR-T cells targeting CD19 and/or CD22;
FIG. 24 illustrates survival curves of mice depicting the *in vivo* anti-double tumor immune escape activity of four types of CAR-T cells targeting CD19 and/or CD22;
FIG. 25 presents imaging results of mice showing the *in vivo* anti-triple tumor immune escape activity of four types of CAR-T cells targeting CD19 and/or CD22;
FIG. 26 presents fluorescence quantitative results of the *in vivo* anti-triple tumor immune escape activity of four types of CAR-T cells targeting CD19 and/or CD22;
FIG. 27 illustrates survival curves of mice depicting the *in vivo* anti-triple tumor immune escape activity of four types of CAR-T cells targeting CD19 and/or CD22;
FIG. 28 demonstrates tumor inhibition activities of four types of CAR-T cells targeting CD19 and/or CD22 in an early PDX model;
FIG. 29 illustrates survival curves of mice of four types of CAR-T cells targeting CD19 and/or CD22 in an early PDX model;
FIG. 30 demonstrates tumor inhibition activities of four types of CAR-T cells targeting CD19 and/or CD22 in a late PDX model;
FIG. 31 illustrates survival curves of mice of four types of CAR-T cells targeting CD19 and/or CD22 in a late PDX model;
FIG. 32 illustrates a schematic structural diagram depicting five types of CAR-T cells targeting Her2 and/or recognizing IGF1R;
FIG. 33 presents a schematic diagram illustrating five types of CAR molecular domains targeting Her2 and/or recognizing IGF1R;
FIG. 34 demonstrates the killing activities of five types of CAR-T cells targeting Her2 and/or recognizing IGF1R against different tumor cell lines;
FIG. 35 demonstrates a comparison of the killing activities of five types of CAR-T cells targeting Her2 and/or recognizing IGF1R against MDA-MB-231 tumor cells;
FIG. 36 demonstrates the cytokine release capacities of five types of CAR-T cells targeting Her2 and/or recognizing IGF1R against MDA-MB-231 tumor cells;
FIG. 37 demonstrates a comparison of the expansion numbers of three types of CAR-T cells targeting Her2 and IGF1R and tumor cell lines being cultured for 6 days;
FIG. 38 demonstrates a comparison of the expansion numbers of three types of CAR-T cells targeting Her2 and IGF1R and tumor cell lines being cultured for 10 days;
FIG. 39 demonstrates a comparison of the proliferation capabilities of three types of CAR-T cells targeting Her2 and IGF1R and MDA-MB-231 tumor cells being cultured for 6 days;
FIG. 40 demonstrates a comparison of the proliferation capabilities of three types of CAR-T cells targeting Her2 and IGF1R and MDA-MB-231 tumor cells being cultured for 10 days;
FIG. 41 demonstrates expression analysis of immunosuppressive receptors for five types of CAR-T cells targeting Her2 and/or IGF1R and tumor cells cultured at an effector-target ratio of 3:1;
FIG. 42 demonstrates expression analysis of immunosuppressive receptors for five types of CAR-T cells targeting Her2 and/or IGF1R and tumor cells cultured at an effector-target ratio of 10:1.

### DETAILED DESCRIPTION OF THE EMBODIMENTS

The present disclosure provides a CAR comprising an antigen-binding domain, a connecting peptide (C-peptide), a hinge region, a transmembrane domain, a 4-1BB co-stimulatory signaling domain, and a CD3ζ signaling domain, wherein the antigen-binding domain includes a dual-target recognition molecule.

In the present disclosure, there are no specific limitations on the hinge region and the transmembrane domain, allowing for the use of any conventional options known in the art. Preferably, the hinge region is a CD8 hinge region, and the transmembrane domain is a CD8 transmembrane domain. The dual-target recognition molecule preferably encompasses a fibronectin (Adnectin), an affibody, a lipocalin (Anticalin), a bicyclic peptide, an ankyrin repeat protein (DARPin), a Fyn kinase derivative (Fynomer), a Kunitz domain, an E7 immune protein, a lymphocyte receptor variable region, a single domain antibody, a complete antibody, an antibody fragment, and an aptamer. Antigens or receptors recognized by the dual-target recognition molecule may include any two from CD19, CD20, CD22, CD33, BCMA, CD123, CD133, CD38, CD39, CD138, CD73, CD30, CD7, CD33, CS1, CD56, CD4, CLL1, Lewis Y, CD138, ROR1, Her2/neu, IGF1R, EGFR, Her3, Her4, VEGFR-1, VEGFR-2, VEGFR-3, mesothelin, GPC2, GPC3, CD33/IL3Ra, c-Met, MUC-1, PSMA, CAIX, CEA, PSCA, GD2, glycolipid F77, EGFRvIII, EpCAM, CD70, Claudin 18.2, IL-13, CD133, CD171, FAP, FBP, PD-L1, NYESO-1, and MAGEA3.

In the present disclosure, the C-peptide preferably comprises a G4S Linker, a Long Hinge Lama Linker, and a β-Stranded Loop Linker (BS Loop). The antigen-binding domain preferably consists of an anti-CD19 scFv and an anti-CD22 nanobody, or an anti-Her2 scFv and a ligand capable of recognizing IGF1R.

In the present disclosure, the anti-CD 19 scFv is preferably an anti-CD 19 monoclonal antibody FMC63, and the anti-CD22 nanobody preferably includes anti-CD22 monoclonal antibodies, such as CD22-Nb25, CD22-Nb35, and CD22-Nb45. When the antigen-binding domain includes both the anti-CD19 scFv and the anti-CD22 nanobody, they are preferably sequentially ligated in series with the C-peptide. The ligation can occur with the G4S Linker or the Long Hinge Lama Linker (LHL), with the LHL preferably has the amino acid sequence EPKIPQPQPKPQPQPQPQPQPKPQPKPEP. Ligation using the G4S Linker results in CD19/CD22(Nb25) G4S Tan CAR with an amino acid sequence set forth in SEQ ID NO: 1. Ligation using the LHL results in CD19/CD22(Nb25) LHL Tan CAR with an amino acid sequence set forth in SEQ ID NO: 2.

In the present disclosure, when the antigen-binding domain comprises both the anti-CD19 scFv and the anti-CD22 nanobody, it preferably additionally includes a variable region of the light chain (VL) of the anti-CD19 scFv, the anti-CD22 nanobody, and a variable region of the heavy chain (VH) of the anti-CD 19 scFv, which are sequentially ligated in series with the C-peptide. Alternatively, the VH of the anti-CD19 scFv, the anti-CD22 nanobody, and the VL of the anti-CD19 scFv are sequentially ligated in series with the C-peptide. This series ligation method is a cyclic series connection, wherein the C-peptide used for the series connection is BS Loop, and the preferred forward and reverse amino acid sequences of the BS Loop are EETKKYQS and SYTYNYEK, respectively. When the VL of anti-CD19 scFv, the anti-CD22 nanobody (anti-CD22 monoclonal antibody CD22-Nb25), and the VH of anti-CD 19 scFv are sequentially ligated in series using the BS Loop, a CAR (namely CD19/CD22(Nb25) BS Loop CAR) structure comprises CD19 VL, EETKKYQS, CD22 nanobody, SYTYNYEK, CD19 VH, CD8 hinge region, CD8 transmembrane domain, 4-1BB co-stimulatory signaling domain, and CD3ζ signaling domain. The CD19/CD22(Nb25) BS Loop CAR has an amino acid sequence set forth in SEQ ID NO: 3. When the anti-CD22 nanobody is anti-CD22-Nb35, the CD19/CD22(Nb35) BS Loop CAR has an amino acid sequence set forth in SEQ ID NO: 4; when the anti-CD22 nanobody is anti-CD22-Nb45, the CD19/CD22(Nb45) BS Loop CAR has an amino acid sequence set forth in SEQ ID NO: 5.

In the amino acid sequence of SEQ ID NO: 3 to SEQ ID NO: 5 of the present disclosure, the signal peptide comprise amino acid sequences shown in positions 1-21 of SEQ ID NO: 3 to SEQ ID NO: 5. The VL of CD19 scFv comprises amino acid sequences shown in positions 22-128 of SEQ ID NO: 3 to SEQ ID NO: 5. The Linker comprises amino acid sequences shown in positions 129-136 and 262-269 of SEQ ID NO: 3, positions 129-136 and 261-268 of SEQ ID NO: 4, and positions 129-136 and 262-269 of SEQ ID NO: 5. The CD22 nanobody-Nb25 comprises amino acid sequences shown in positions 137-261 of SEQ ID NO: 3. The CD22 nanobody-Nb35 comprises amino acid sequences shown in positions 137-260 of SEQ ID NO: 4. The CD22 nanobody-Nb45 comprises amino acid sequences shown in positions 137-261 of SEQ ID NO: 5. The VH of the CD19-scFv comprises amino acid sequences shown in positions 270-389 of SEQ ID NO: 3, positions 269-388 of SEQ ID NO: 4, or positions 270-389 of SEQ ID NO: 5. The CD8 hinge region comprises amino acid sequences shown in positions 390-434 of SEQ ID NO: 3, positions 289-433 of SEQ ID NO: 4, or positions 390-434 of SEQ ID NO: 5. The CD8 transmembrane domain comprises amino acid sequences shown in positions 435-458 of SEQ ID NO: 3, positions 434-457 of SEQ ID NO: 4, or positions 435-458 of SEQ ID NO: 5. The 4-1BB co-stimulation signaling domain comprises amino acid sequences shown in positions 459-500 of SEQ ID NO: 3, positions 458-499 of SEQ ID NO: 4, or positions 459-500 of SEQ ID NO: 5. The CD3ζ signaling domain comprises amino acid sequences shown in positions 501-612 of SEQ ID NO: 3, positions 500-611 of SEQ ID NO: 4, or positions 501-612 of SEQ ID NO: 5.

In the present disclosure, the anti-Her2 scFv preferably comprises the anti-Her2 monoclonal antibody 4D5, and the ligand capable of recognizing IGF1R is preferably Adnectin. When the antigen-binding domain consists of the anti-Her2 scFv and the IGF1R-recognizing ligand Adnectin, it is preferred that the antigen-binding domain comprises the IGF1R-recognizing ligand and the anti-Her2 scFv sequentially ligated in series, or the anti-Her2 scFv and the IGF1R-recognizing ligand sequentially ligated in series. In cases where the IGF1R-recognizing ligand and the anti-Her2 scFv, or vice versa, are sequentially ligated in series, the C-peptide is preferably the G4S Linker and the β-Stranded Loop Linker, with the G4S Linker having an amino acid sequence of (GGGGS)ₙ (n≥1).

When the G4S Linker C-peptide is utilized to sequentially ligate the IGF1R-recognizing ligand Adnectin and the anti-Her2 scFv, the resulting constructs is referred to as the IGF1R/Her2 Tan CAR, having an amino acid sequence set forth in SEQ ID NO: 10. Conversely, when the G4S Linker C-peptide is employed to sequentially ligate the anti-Her2 scFv and the IGF1R-recognizing ligand Adnectin, the resulting construct is denoted as the Her2/IGF1R Tan CAR, with an amino acid sequence set forth in SEQ ID NO: 11.

When the antigen-binding domain comprises the anti-Her2 scFv and the IGF1R-recognizing ligand Adnectin, it is preferably extended to include the VL of anti-Her2 scFv (4D5 V_{L}), the IGF1R-recognizing ligand, and the VH of anti-Her2 scFv (4D5 V_{H}), all sequentially ligated in series with the C-peptide. Alternatively, it may include the VH of the anti-Her2 scFv (4D5 V_{H}), the IGF1R-recognizing ligand, and the VL of the anti-Her2 scFv (4D5 V_{L}), sequentially ligated in series with the C-peptide. When the BS Loop C-peptide is employed to sequentially ligate the VL of the anti-Her2 scFv (4D5 V_{L}), the IGF1R-recognizing ligand, and the VH of the anti-Her2 scFv (4D5 V_{H}), the resulting construct is termed the Her2/IGF1R BS Loop CAR, having an amino acid sequence set forth in SEQ ID NO: 12.

In the amino acid sequences of SEQ ID NO: 10 to SEQ ID NO: 12 of the present disclosure, the signal peptide is represented by amino acid sequences shown in positions 1-21 of SEQ ID NO: 10 to SEQ ID NO: 12. The VL of 4D5 scFv is delineated by amino acid sequences shown in positions 126-234 of SEQ ID NO: 10 and positions 22-130 of SEQ ID NO: 11 to SEQ ID NO: 12. The Linker comprises amino acid sequences shown in positions 131-138 and 238-245 of SEQ ID NO: 12. The IGF1R-binding ligand Adnectin encompasses amino acid sequences shown in positions 22-120 of SEQ ID NO: 10, positions 274-372 of SEQ ID NO: 11, and positions 139-237 of SEQ ID NO: 12. The VH of 4D5-scFv is illustrated by amino acid sequences shown in positions 250-372 of SEQ ID NO: 10, positions 146-268 of SEQ ID NO: 11, and positions 246-368 of SEQ ID NO: 12. The CD8 hinge region is identified by amino acid sequences shown in positions 373-417 of SEQ ID NO: 10 to SEQ ID NO: 11 and positions 369-413 of SEQ ID NO: 12. The CD8 transmembrane domain is indicated by amino acid sequences shown in positions 418-441 of SEQ ID NO: 10 to SEQ ID NO: 11 and positions 414-437 of SEQ ID NO: 12. The 4-1BB co-stimulatory signaling domain is denoted by amino acid sequences shown in positions 442-483 of SEQ ID NO: 10 to SEQ ID NO: 11 and positions 438-479 of SEQ ID NO: 12. The CD3ζ signaling domain encompasses amino acid sequences shown in positions 438-479 of SEQ ID NO: 10 to SEQ ID NO: 11 and positions 480-591 of SEQ ID NO: 12.

In a specific example of the present disclosure, a single-target IGF1R CAR and a Her2 CAR are employed as primary comparative examples, wherein the IGF1R CAR is delineated in an amino acid sequence set forth in SEQ ID NO: 9; and the Her2 CAR is outlined in an amino acid sequence set forth in SEQ ID NO: 13. The signal peptide encompasses amino acid sequences shown in positions 1-21 of SEQ ID NO: 13. The VL of 4D5 scFv is depicted by amino acid sequences shown in positions 22-130 of SEQ ID NO: 13. The IGF1R-binding ligand Adnectin is represented by amino acid sequences shown in positions 22-120 of SEQ ID NO: 9. The VH of 4D5-scFv is characterized by amino acid sequences shown in positions 146-268 of SEQ ID NO: 13. The CD8 hinge region is identified by amino acid sequences shown in positions 121-165 of SEQ ID NO: 9 or positions 121-165 of SEQ ID NO: 13. The CD8 transmembrane domain is denoted by amino acid sequences shown in positions 166-189 of SEQ ID NO: 9 or positions 166-189 of SEQ ID NO: 13. The 4-1BB co-stimulatory signaling domain is indicated by amino acid sequences shown in positions 190-231 of SEQ ID NO: 9 or positions 190-231 of SEQ ID NO: 13. The CD3ζ signaling domain encompasses amino acid sequences shown in positions 232-343 of SEQ ID NO: 9 or positions 232-343 of SEQ ID NO: 13.

The present disclosure further provides a recombinant lentivirus, wherein the recombinant lentivirus is obtained by co-transfection of a viral vector containing the CAR with a mammalian cell.

In the present disclosure, the mammalian cell is preferably a 293 cell, a 293T cell, or a 293FT cell, with the 293FT cell being the most preferred. The viral vector carrying the CAR preferably adopts a three-plasmid system or a four-plasmid system, with the latter being more preferable. The four-plasmid system comprises a lentivirus expression plasmid carrying the target gene, two packaging plasmids, and a plasmid encoding an envelope protein, thereby enhancing the stability and safety of the viral vector.

The present disclosure further provides a CAR-T cell, wherein the CAR-T cell expresses the CAR; or the genome of the CAR-T cell integrates the amino acid sequences of the CAR; or the CAR-T cell includes the recombinant lentivirus.

In the present disclosure, there is no specific limitation on the preparation method of the CAR-T cells, and any conventional method known in the art can be employed. In a specific example, the preparation method includes the following steps: cloning a target gene fragment into a pLV vector to obtain a pLV-CD19/CD22(Nb25) BS Loop CAR or pLV-Her2/IGF1R BS Loop CAR, wherein a BS Loop sequence is incorporated into a primer sequence for amplifying CD22 nanobody and anti-IGF1R (Adnectin); co-transfecting the viral vector containing CAR and the mammalian cell using Lipofectamine^{™} 2000 transfection reagent to generate a lentivirus expressing BS Loop CAR; infecting activated T cells with the lentivirus and transfecting with polybrene transfection reagent to obtain CAR-T cells expressing BS Loop CAR.

The present disclosure further provides use of the CAR, the amino acid sequence, the recombinant lentivirus, or the CAR-T cell in preparation of a drug. Preferably, the drug comprises an anti-tumor immunotherapy drug, and the diseases treated by the drugs include CD19-positive and/or CD22-positive diseases as well as Her2-positive and/or IGF1R-positive diseases.

The technical solution provided by the present disclosure will be elaborated upon in detail with reference to the examples provided below, but they should not be construed as limiting the claimed scope of the present disclosure. In the following examples, reagents and consumables used in the experiments can be purchased or prepared following existing disclosed technologies; those without specified sources and specifications are commercially available; various procedures and methods not explicitly described are conventional methods well known in the art.

### Example 1

### Preparation of CD19/CD22 BS Loop CAR-T cells

### Preparation of CD19/CD22(Nb25) BS Loop CAR-T (SEQ ID NO: 3) cells

To create the desired constructs, the inventor started with commercially available CD19 CAR-T cells (Kymriah), and extracted the CD19 scFv V_{L} region. This was amplified using a forward primer with the sequence ggtaccgcggccgcccggggatccatggccttaccagtgaccgccttgctcctgccg and a reverse primer with the sequence tagtctcctctgtgatctccagcttggtcc. Next, the CD22 nanobody fragment was amplified using a forward primer with the sequence aagctggagatcacaGAGGAGACTAAAAAATATCAGTCTgaagtgcaactggtggaatc and a reverse primer with the sequence ctgcagtttcacctctttttcgtaattataagtgtaTCTTACACTTATAATTACGAAAAAagaagaactcacagtcacctgg g. Additionally, the CD19 scFv V_{H} region was amplified using a forward primer with the sequence ttacgaaaaagaggtgaaactgcaggagtc and a reverse primer with the sequence caagggcgcccagatgtagatatcacaggcgaagtccagccccctcgtgtg. These three PCR products were then recombined into a target gene fragment through overlapping PCR. The sequences for the CD19/CD22 BS Loop CAR are included in uppercase in the primer sequences (in uppercase). The PCR setup and reaction conditions are detailed in Table 1 and Table 2, respectively.

**Table 1: PCR system**

| **Reagent** | **Volume** |
|---|---|
| PrimeSTAR MAX DNA Premix | 25 µL |
| Forward primer (10 pmol/µL) | 1 µL |
| Reverse primer (10 pmol/µL) | 1 µL |
| DNA Template | 1 µL |
| ddH₂O | 22 µL |
| Total system 51 µL | |

**Table 2: PCR conditions**

| **Step** | | **Temperature** | **Time** |
|---|---|---|---|
| Initial denaturation | | 95°C | 4 min |
| 30 cycles | Denaturation | 94°C | 15 sec |
| | Annealing | 55°C | 15 sec |
| | Extension | 72°C | 1 min |
| Extension | | 72°C | 7 min |
| Storage | | 4°C | ∞ |

After the PCR reaction, the resulting PCR products were transferred into 1.5 mL centrifuge tubes and mixed with 5 times their volume of CP Buffer. After thorough mixing, the mixture was loaded onto a centrifugal adsorption column, and centrifuged at 14,000 g for 1 minute at room temperature. Next, a Wash Buffer was added, and the column was centrifuged again at 14,000 g for 1 minute. The adsorption column was then transferred to a new 1.5 mL centrifuge tube, and 50 µL of sterilized deionized water was carefully pipetted onto the center of the column. After incubating at room temperature for 2 minutes, the column was centrifuged to elicit the purified products, enabling the quantification of the DNA concentration. Subsequently, the purified products were stored at -20 °C. For the vector preparation, it was subjected to overnight digestion at 37 °C using the enzymes specified in Table 3. After digestion, the samples were resolved by loading onto a 2% agarose gel for electrophoresis, followed by gel extraction to isolate the desired DNA fragments.

**Table 3: Enzyme digestion system**

| **Reagent** | **Volume** |
|---|---|
| pLv vector | 3 µg |
| *BamH*I | 2 µL |
| *Sal*I | 2 µL |
| 10× CutSmart Buffer | 5 µL |
| Total system 50 µL | |

The digested vector and PCR products containing the target sequence were mixed at a molar ratio of 1:2, followed by the addition of a recombinase enzyme. After incubation at 50°C for 5 minutes, the mixture was promptly transferred to ice to prepare for transformation. The resulting recombinant vector, designated as pLV-CD19/CD22 BS Loop CAR, was obtained after recombination facilitated by the recombinase enzyme.

### Plasmid transformation

Competent cells were removed from a -80 °C freezer and thawed on ice. The plasmid was added to the competent cells and mixed thoroughly by gently tapping the tube with finger. The mixture was then incubated on ice for 10 minutes. The cells underwent heat shock in a 42 °C water bath for 45 seconds, then were promptly transferred back to ice for 2 minutes. Following this, the cells were mixed with SOC medium and incubated at 37 °C with shaking at 150 rpm for 1 hour to allow recovering. Afterward, 100 µL of the cell suspension was plated onto LB agar containing ampicillin and incubated overnight at 37 °C. Single colonies were selected for sequencing, and plasmids were extracted from colonies with the correct sequence.

### Plasmid Maxiprep:

Pelleting the bacteria: The overnight bacterial culture was centrifuged at 8,000 rpm for 15 minutes, and the supernatant was discarded completely.

Resuspension: All bacterial pellets were resuspended in 5 mL of a cell suspension solution containing RNase A.

Lysis: A cell lysis solution was added, and the tube was inverted several times to ensure complete cell lysis.

Neutralization: A neutralization buffer was added, and the tube was gently inverted several times to mix thoroughly, followed by centrifugation to remove debris.

Precipitation: The supernatant was transferred to a new container, mixed with isopropyl alcohol, centrifuged, and the resulting precipitate was washed with 75% ethanol before air drying.

RNA Removal: The precipitate was dissolved in a solution containing RNase A and transferred to a 1.5 mL centrifuge tube.

Impurity Removal: An impurity removal solution was added, the tube was inverted to mix thoroughly, and then centrifuged. The supernatant was discarded.

Second Precipitation: The remaining precipitate was dissolved in a high-salt solution, mixed with anhydrous ethanol, allowed to stand, then centrifuged to isolate the plasmid DNA. The supernatant was removed.

Washing: The precipitate was washed with 75% ethanol and centrifuged to remove impurities.

Drying: The open centrifuge tube was left in a laminar flow hood to allow complete evaporation of ethanol.

Dissolution: Deionized water was added to dissolve the plasmid DNA, and the dissolved sample was stored at -20 °C for future use.

### PBMC extraction

An appropriate volume of Ficoll was added in a centrifuge tube. Whole blood was slowly layered over the Ficoll in the centrifuge tube, allowing the separation of components by density. The mixture was centrifuged at 2,000 rpm for 30 minutes at 27 °C. Following centrifugation, three distinct layers formed: the upper layer contained serum, the middle layer was the Ficoll, and the bottom layer comprised red blood cells and granulocytes. A white band, representing PBMCs, formed between the upper and middle layers. The white band was carefully aspirated with a Pasteur pipette and transferred to a new centrifuge tube. Approximately 5-fold volume of DPBS was added to the tube, mixed thoroughly by inversion, and then centrifuged at 2,000 rpm for 10 minutes at 27 °C to wash the PBMCs. The supernatant was discarded, and the cells were resuspended in 5 mL of RPMI-1640 medium. The suspension was transferred to T25 culture flasks and incubated at 37 °C for 1 hour to allow the adherent cells to attach to the surface. The non-adherent cells were then counted and frozen at a concentration of 1×10⁸ cells per 100 µL in each storage tube.

### PBMC resuscitation and activation

Thawed PBMCs were added to 10 mL of AIM V medium, then centrifuged at 1,500 rpm for 5 minutes at 27 °C. After discarding the supernatant, the PBMCs were resuspended in AIM-V medium for cell counting. CD3/CD28 magnetic beads were prepared by washing them once with 2 mL of PBS containing 5% FBS, followed by an additional wash with 2 mL of AIM V medium. A total of 1×10⁶ T cells were collected and combined with the magnetic beads and AIM V medium, with the addition of IL-2 to reach a final concentration of 300 IU/mL. The mixture was then incubated in a 24-well plate for 48 hours to activate the T cells.

### Preparation of lentivirus

The 293FT cells were prepared at a density of 0.75×10⁶ cells/mL and seeded at 2 mL per well in a 24-well plate. After 48 hours, when the adherent 293FT cells reached about 90% confluency, a transfection mixture containing lentiviral plasmids and Lipofectamine^{™} 2000 was prepared. This was done by combining transfection reagents A and B, then letting the mixture sit at room temperature for 30 minutes. The transfection mixture was then gently added to the corresponding wells in a clockwise manner. After an additional 48 hours, the viral supernatant was collected by centrifuging to remove cell debris. This supernatant contained the lentivirus particles for future use.

### Lentivirus-mediated transduction of T cells

Forty-eight hours after PBMC activation, the cells were counted to determine cell density. A cell suspension was prepared in each well, and lentivirus was added at the desired concentration along with IL-2 to achieve a final concentration of 300 IU/mL. Polybrene was also added at a final concentration of 6 µg/mL to enhance transduction efficiency. The plate was centrifuged at 35 °C for 90 minutes at 1,000 g, then transferred to a 37 °C cell culture incubator. After 15 hours of transduction, the medium was replaced with fresh AIM V medium containing IL-2 at a final concentration of 300 IU/mL, and the cells were returned to the 24-well plate. After 2-4 days, when the medium began to turn yellow, indicating cell growth and metabolism, the cells were transferred to a 6-well plate to further expansion with fresh medium. The beads were removed after 7 days of transduction.

### Example 2

### Fluorescent labeling of CD19 and CD22 antigens and detection of CAR-T cell expression

The antigen protein was concentrated to between 0.5 mg/mL to 1 mg/mL, then mixed with a modifier reagent. The mixture was gently transferred to a container with APC or PE dye dry powder and gently mixed. It was then left at room temperature in the dark for 3 hours. The reaction was terminated by adding a quenching reagent, and the resulting labeled antigen protein was set aside for further use.

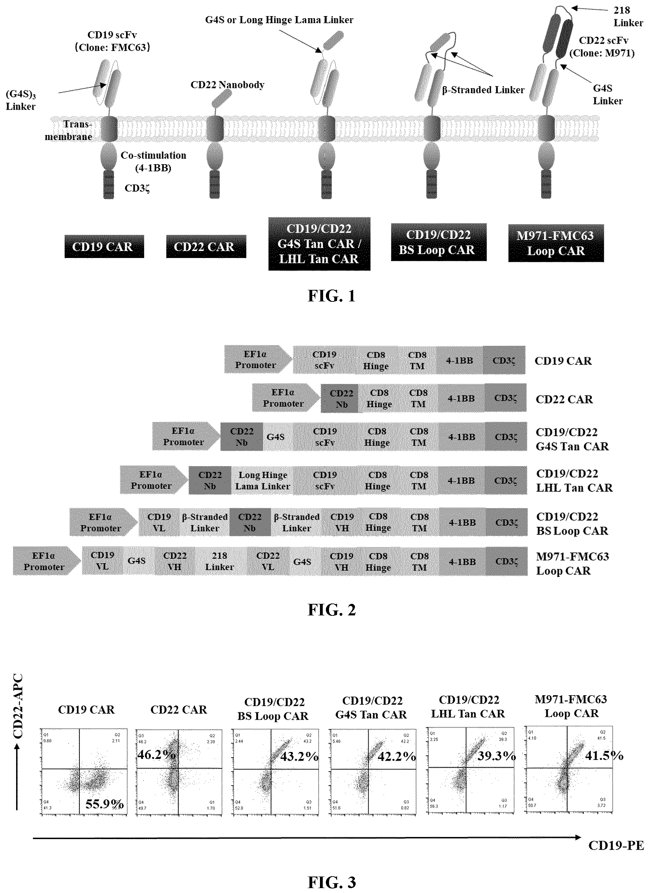

CD19-APC and CD22-PE antigens, each at a concentration of 100 nM, were incubated on ice with six different types of CAR-T cells, including: CD19 CAR-T (with the amino acid sequence described in SEQ ID NO: 6), CD22 CAR-T (CD22(Nb25), with the amino acid sequence in SEQ ID NO: 7), CD19/CD22 BS Loop CAR-T (as defined in SEQ ID NO: 3), CD19/CD22 G4S Tan CAR-T (as defined in SEQ ID NO: 1), CD19/CD22 LHL Tan CAR-T (as defined in SEQ ID NO: 2), and M971-FMC63 Loop CAR-T (as defined in SEQ ID NO: 8). The structural and molecular diagrams of the six types of CARs were detailed in FIG. 1 and FIG. 2, respectively. The CAR-T cells were resuspended in FACS buffer and then incubated with the CD19-APC and CD22-PE antigens. After 1 hour, the cells were washed twice with PBS and resuspended in PBS for analysis by flow cytometry to assess the binding of CAR-T cells to the labeled antigens. The results of the analysis are presented in FIG. 3.

As shown in FIG. 3, the CD19 CAR-T cells could bind only to the CD19 antigen, and the CD22 CAR-T cells could bind only to the CD22 antigen. However, all four types of bispecific CAR-T cells (CD19/CD22 BS Loop CAR-T, CD19/CD22 G4S Tan CAR-T, CD19/CD22 LHL Tan CAR-T, and M971-FCM63 Loop CAR-T) could bind to both CD19 and CD22 antigens. Among these bispecific CAR-T cells, the CD19/CD22 BS Loop CAR-T exhibited the highest binding efficiency.

### Example 3

### Analysis of tumor cell killing by CD19/CD22 dual-target CAR-T cells with different structures

Tumor cells: Nalm6-GL, Nalm6-GL-KO19, Nalm6-GL-KO22, K562-CD19, K562-CD22, K562 or primary tumor cells from four B-cell ALL patients.

CAR-T cells: The three types of CD19/CD22 bispecific CAR-T cells from Example 2 were used: CD19/CD22 BS Loop CAR-T (amino acid sequence in SEQ ID NO: 3), CD19/CD22 G4S Tan CAR-T (as defined in SEQ ID NO: 1), and the CD19/CD22 LHL Tan CAR-T (as defined in SEQ ID NO: 2). Prior to the assay, these CAR-T cells were deprived of IL-2 for 24 hours to ensure a starvation state.

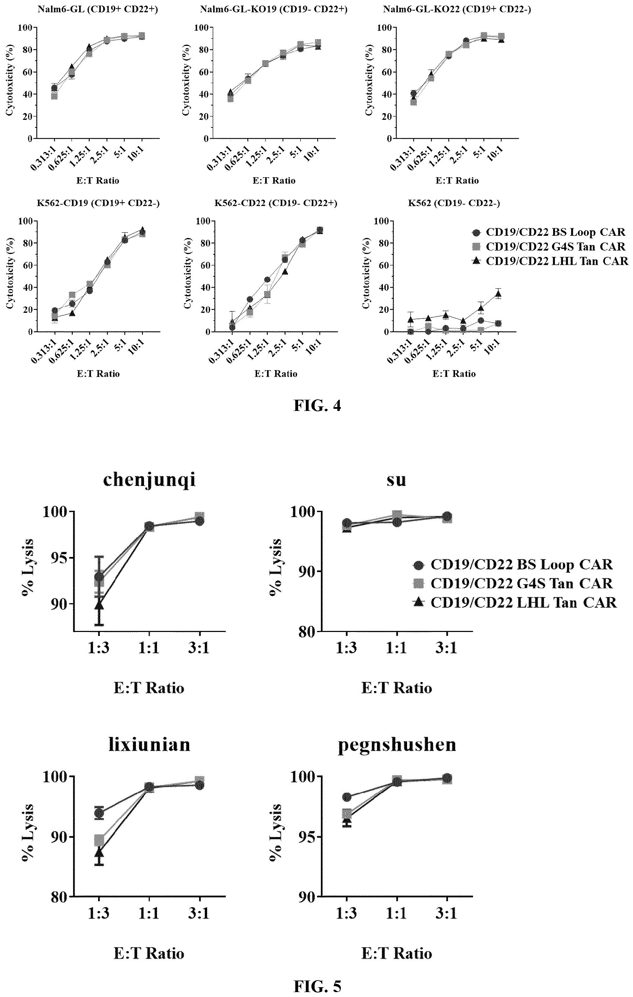

The specified CAR-T cells were co-cultured with tumor cells at various effector-target ratio (E: T), including 10:1, 5:1, 2.5:1, 1.25:1, 0.625:1, 0.3125:1, as well as 1:3, 1:1, 3:1.
1) Cell Counting: Tumor cells and CAR-T cells were resuspended in RPMI-1640 medium and counted. If the tumor cells were difficult to resuspend or prone to clumping, the culture was allowed to settle, and the supernatant was used for cell counting.
2) CFSE Staining: 5×10⁶ tumor cells were stained in a 10 mL centrifuge tube. The cells were resuspended in 0.5 mL of staining solution at a concentration of 10⁷ cells/mL and stained at 37 °C for 15 minutes. FBS was added for neutralization, followed by additional RPMI-1640 medium to reach 4 mL, centrifuged to remove the supernatant, the precipitate was resuspended in 10 mL of medium and centrifuged to wash 3 times in total.
3) Cell Dilution: The CAR-T cells were diluted to 2×10⁶ cells/mL. A total of 1.5 mL of this cell suspension was pipetted into a 24-well plate, followed by sequential dilution to achieve gradients of 1×10⁶ cells/mL, 0.5×10⁶ cells/mL, 0.25×10⁶ cells/mL, 0.125×10⁶ cells/mL, and 0.0625×10⁶ cells/mL, with 500 µL of each diluted sample used in the assay.
4) Additional Preparations: An additional 2 mL of stained and unstained tumor cells were separately cultured in a 24-well plate for flow cytometry calibration and voltage adjustment.
5) Flow Cytometry Setup: To set up the flow cytometer, 1 mL of the unstained tumor cells from the 24-well plate was collected per tube. These samples were treated with 7-AAD, UV-exposed for 30 minutes, and then incubated at 95 °C for 10 minutes to create positive controls. Another set of 1 mL of unstained tumor cells was used for flow cytometry baseline adjustment. Meanwhile, the CFSE-stained tumor cells were used to set the flow cytometry voltage. A 7-AAD solution was prepared by diluting 280 µL in 28 mL PBS and mixed thoroughly, with 100 µL added to each well of a 96-well plate. The samples were incubated on a shaker for 30 minutes for analysis.
6) Flow Cytometry Analysis: To determine the killing efficiency, the number of viable tumor cells were counted. Viable tumor cells were identified as CFSE-positive and 7-AAD-negative. The killing efficiency was calculated as: % Lysis = (number of viable tumor cells in control group - number of viable tumor cells in experimental group)/number of viable tumor cells in control group ×100. The results of analysis are shown in FIG. 4 and FIG. 5. As depicted in FIG. 4, among the three bispecific CAR-T cells, the CD19/CD22 G4S Tan CAR-T and CD19/CD22 BS Loop CAR-T demonstrated similar tumor-killing activity, while CD19/CD22 LHL Tan CAR-T exhibited greater non-specific killing activity against negative control cells such as K562. In addition, as shown in FIG. 5, the CD19/CD22 BS Loop CAR-T demonstrated a stronger tumor-killing activity against the primary tumor cells from B-cell ALL patients.

### Example 4

### Analysis of tumor cell killing by CD19/CD22 BS Loop CAR-T and single-target CAR-T

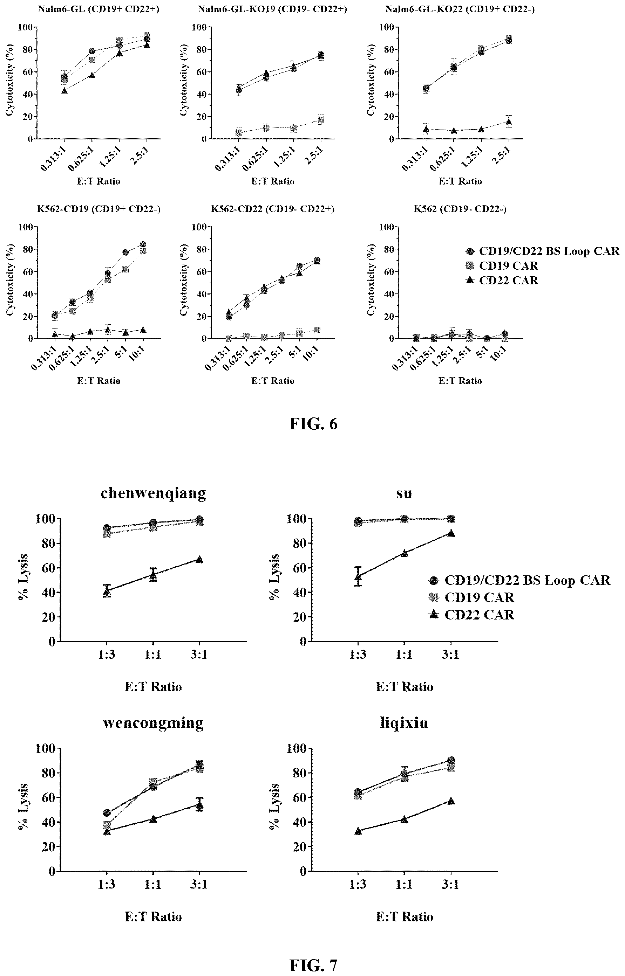

This example followed the method described in Example 3, with the following differences: the CAR-T cells used were the CD19/CD22 BS Loop CAR-T (amino acid sequence in SEQ ID NO: 3), CD19 CAR-T (as defined in SEQ ID NO: 6), and CD22 CAR-T (CD22(Nb25), as defined in SEQ ID NO: 7); Additionally, the primary tumor cells were obtained from four different patients with B-cell ALL. The results are illustrated in FIG. 6 and FIG. 7.

As shown in FIG. 6, the analysis of tumor cell killing revealed that the CD19 CAR-T could only kill CD19-positive tumor cells, and the CD22 CAR-T could only kill CD22-positive tumor cells. In contrast, the CD19/CD22 BS Loop CAR-T was capable of killing both CD19 and/or CD22-positive tumor cells. Significantly, when targeting CD19/CD22 double-positive Nalm6 tumor cells and CD19 positive K562-CD19 tumor cells, the CD19/CD22 BS Loop CAR-T exhibited higher killing activity compared to the single-target CAR-T cells. Similarly, FIG. 7 indicated that the CD19/CD22 BS Loop CAR-T demonstrated greater effectiveness in killing the primary tumor cells from patients compared to the single-target CAR-T cells, than that of single-target CAR-T cells.

### Example 5

### Analysis of tumor cell killing by CD19/CD22 BS Loop CAR-T and reported CD19/CD22 CAR-T

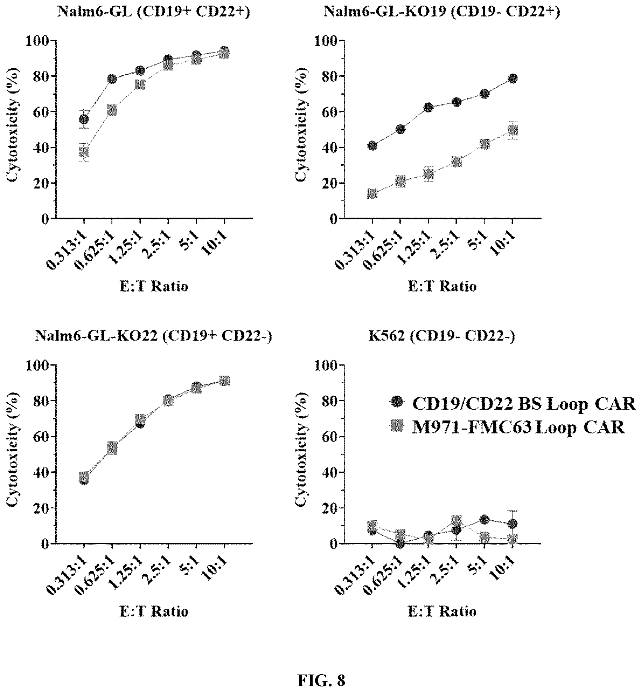

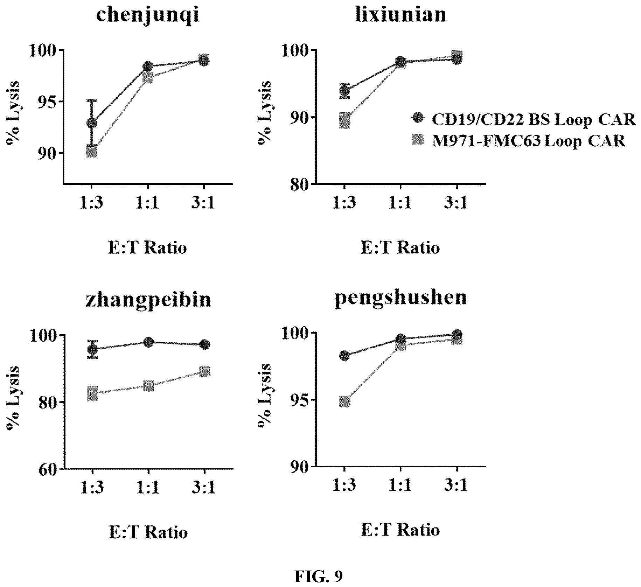

This example followed the method described in Example 3, with the following differences: the CAR-T cells used were the CD19/CD22 BS Loop CAR-T (amino acid sequence in SEQ ID NO: 3) and M971-FMC63 Loop CAR-T (as defined in SEQ ID NO: 8) from Example 2. The tumor cells used for this analysis were Nalm6-GL, Nalm6-GL-KO19, Nalm6-GL-KO22, K562, or primary tumor cells from four patients with B-cell ALL. The results of the analysis are detailed in FIG. 8 and FIG. 9.

As illustrated in FIG. 8 and FIG. 9, the CD19/CD22 BS Loop CAR-T cells of the present disclosure demonstrated greater tumor cell killing activity against both the tumor cell lines and primary tumor cells from patients compared to the previously reported circularly connected M971-FMC63 Loop CAR-T cells.

### Example 6

### Analysis of cytokine release by CD19/CD22 dual-target CAR-T with different structures

Tumor Cells: Nalm6, Nalm6-KO19, Nalm6-KO22, K562-CD19, K562-CD22, K562 or primary tumor cells from four patients with B-cell ALL.

CAR-T Cells: The three types of CD19/CD22 bispecific CAR-T cells described in Example 2 were used: CD19/CD22 BS Loop CAR-T (amino acid sequence in SEQ ID NO: 3), CD19/CD22 G4S Tan CAR-T (as defined in SEQ ID NO: 1), and CD19/CD22 LHL Tan CAR-T (as defined in SEQ ID NO: 2). Prior to the experiment, the CAR-T cells were deprived of IL-2 for 24 hours.

The CAR-T cells were co-cultured with tumor cells at an effector-target ratio (E: T) of 1:3.

Following centrifugation to remove the supernatant, the CAR-T cells were resuspended, counted, and diluted to a concentration of 0.2×10⁶ cells/mL. Tumor cells were treated similarly, diluted to a concentration of 0.6×10⁶ cells/mL, then combined with the CAR-T cells at equal volumes and mixed thoroughly. After co-culturing for 24 hours, the supernatant was collected and stored at -20 °C for later cytokine analysis.

The levels of IL-2, IFN-γ, and TNF-α were measured using ELISA detection kits. The process involved coating the plate with Capture Antibody, allowing it to stand at room temperature for 4 hours or overnight at 4 °C. After washing with Wash Buffer, the plate was blocked with ELISA/ELISAPOT Diluent (1×) for 2 hours at room temperature or overnight at 4 °C. The standards for IL-2, IFN-γ, and TNF-α were prepared and added to the plate, along with diluted CAR-T cell/tumor cell supernatant. After incubation at 37 °C for 2 hours and subsequent washing, Detection Antibody was added, and incubated at room temperature for 1-2 hours; the plate was washed another 5 times with Wash Buffer, added with Streptavidin-HRP solution, allowed to stand at room temperature for 30 minutes to 1 hour, and then the plate was washed 5 times. 1× TMD solution was added for color development, and Stop Solution was used to terminate the reaction. The absorbance at a wavelength of 450nm was measured using the microplate reader.

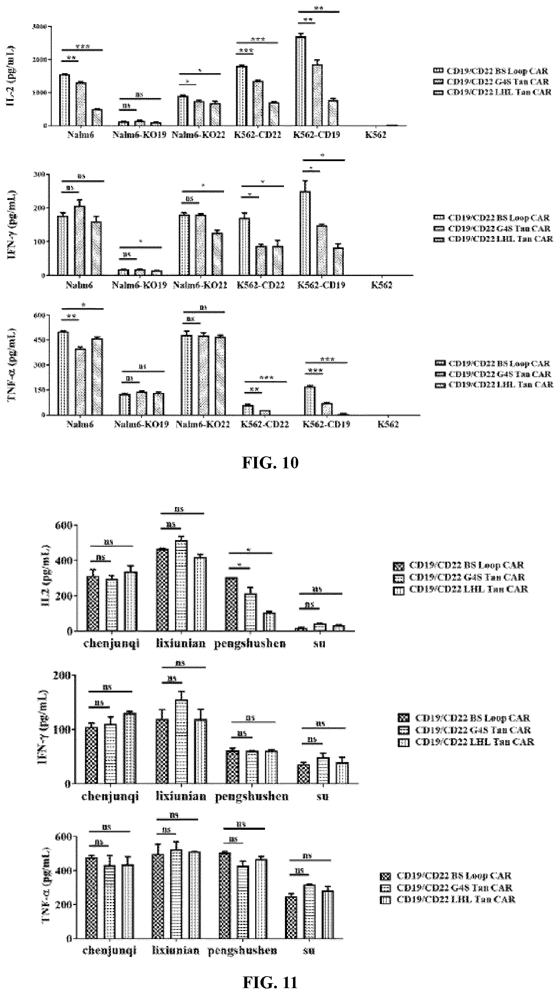

The results are detailed in FIG. 10 and FIG. 11. As shown in FIG. 10, the CD19/CD22 BS Loop CAR-T of the present disclosure exhibited higher cytokine release levels compared to the CD19/CD22 G4S Tan CAR-T and CD19/CD22 LHL Tan CAR-T against CD19- and/or CD22-positive tumor cell lines. FIG. 11 focused on cytokine release in response to primary tumor cells, indicating that the CD19/CD22 BS Loop CAR-T of the present disclosure produced higher cytokine levels when tested against primary tumor cells from a patient named pengshushen.

### Example 7

### Analysis of cytokine release by CD19/CD22 BS Loop CAR-T and single-target CAR-T

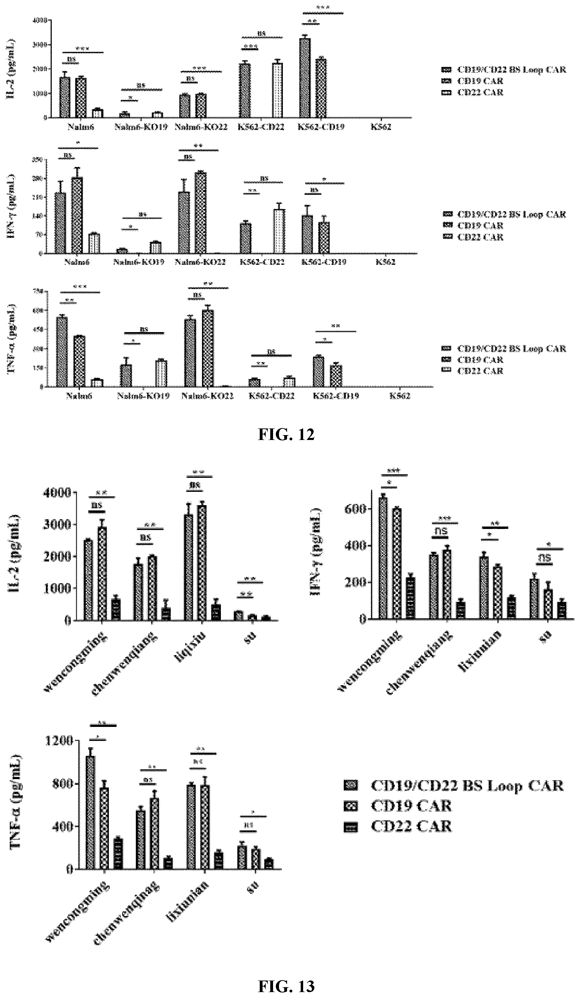

The example was conducted according to the method described in Example 6, with the following modification: the CAR-T cells were the CD19/CD22 BS Loop CAR-T (amino acid sequence in SEQ ID NO: 3), CD19 CAR-T (as defined in SEQ ID NO: 6), and CD22 CAR (CD22(Nb25), as defined in SEQ ID NO: 7) from Example 2. The results are detailed in FIG. 12 to FIG. 13.

As shown in FIG. 12, the CD19/CD22 BS Loop CAR-T of the present disclosure demonstrated the ability to release cytokines when co-cultured with tumor cells that are positive for CD19 and/or CD22. Furthermore, the CD19/CD22 BS Loop CAR-T exhibited a higher level of cytokine release compared to the single-target CAR-T cells. Similarly, as shown in FIG. 13, the CD19/CD22 BS Loop CAR-T of the present disclosure showed a higher level of cytokine release against primary patient tumor cells when compared to single-target CAR-T cells.

### Example 8

### Analysis of cytokine release by CD19/CD22 BS Loop CAR-T and reported CD19/CD22 CAR-T

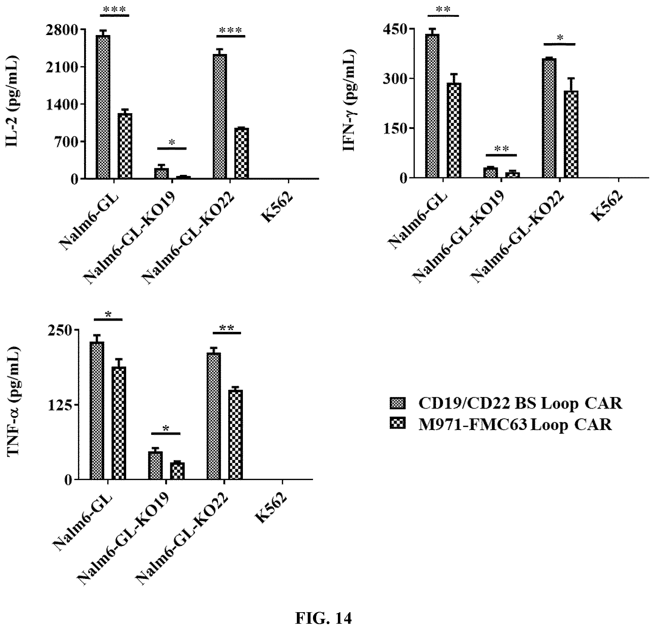

This example followed the method described in Example 6, with the following variation: the CAR-T cells used were the CD19/CD22 BS Loop CAR-T (amino acid sequence in SEQ ID NO: 3) and M971-FMC63 Loop CAR-T (as defined in SEQ ID NO: 8) from Example 2. The tumor cells employed for this analysis were Nalm6-GL, Nalm6-GL-KO19, Nalm6-GL-KO22, K562, and primary tumor cells from four B-cell ALL patients. The results are detailed in FIG. 14 through FIG. 15.

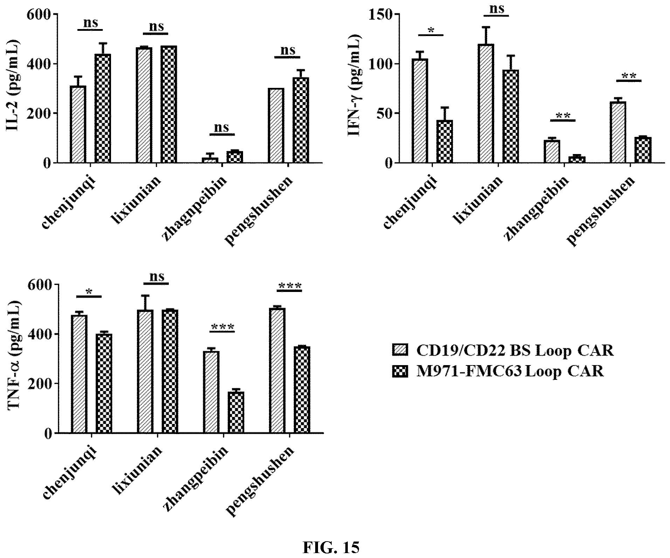

As shown in FIG. 14 and FIG. 15, the CD19/CD22 BS Loop CAR-T cells demonstrated a higher level of cytokine release when co-cultured with tumor cell lines and primary patient tumor cells compared to the M971-FMC63 Loop CAR-T cells.

### Example 9

### Analysis of activation by CD19/CD22 dual-target CAR-T cells with different structures

Tumor Cells: Nalm6, K562-CD19, K562-CD22, and K562.

CAR-T Cells: The three types of CAR-T cells mentioned in Example 2 were tested: CD19/CD22 BS Loop CAR-T (amino acid sequence in SEQ ID NO: 3), CD19/CD22 G4S Tan CAR-T (as defined in SEQ ID NO: 1), and the CD19/CD22 LHL Tan CAR-T (as defined in SEQ ID NO: 2). These CAR-T cells were starved to remove IL-2 24 hours in advance.

The CAR-T cells were co-cultured with tumor cells at an effector-target ratio (E: T) of 1:3.

After centrifuging to remove the supernatant, the CAR-T cells were resuspended, counted and diluted to a concentration of 0.2×10⁶ cells/mL. Tumor cells were centrifuged and resuspended, then diluted to 0.6×10⁶ cells/mL. Equal volumes of CAR-T cells and tumor cells were mixed and co-cultured for 24 hours. These cells were then used to detect CD25 expression. Flow cytometry was performed by staining with CD25-FITC antibody on ice for 1 hour, followed by two washes with FACS buffer and then resuspension for flow cytometry analysis.

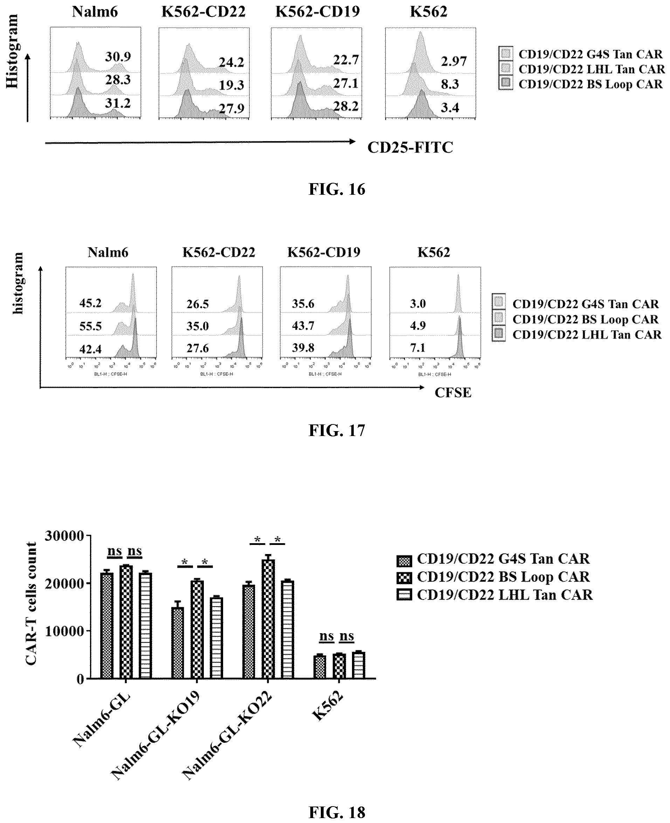

The activation results are shown detailed in FIG. 16. As indicated, all three types of bispecific CAR-T cells expressed CD25, the activation marker, after co-culturing with the target cells. Notably, the CD19/CD22 BS Loop CAR-T exhibited the highest percentage of CD25 expression, indicating the greatest level of activation among the tested CAR-T cells.

### Example 10

### Analysis of proliferation rate by CD19/CD22 dual-target CAR-T cells with different structures

Tumor cells: Nalm6, K562-CD19, K562-CD22, and K562.

CAR-T cells: Three types of CAR-T cells described in Example 2 were used: CD19/CD22 BS Loop CAR-T (amino acid sequence set forth in SEQ ID NO: 3), CD19/CD22 G4S Tan CAR-T (amino acid sequence set forth in SEQ ID NO: 1), and the CD19/CD22 LHL Tan CAR-T (amino acid sequence set forth in SEQ ID NO: 2), were starved to remove IL-2 24 hours in advance.

The CAR-T cells were co-cultured with tumor cells at an effector-target ratio (E: T) of 1:3.

The CAR-T cells were labeled using a CFSE fluorescent labeling kit and adjusted to a density of 0.2×10⁶ cells/mL. The supernatant was removed by centrifugation, and the tumor cells were resuspended, counted, and diluted to 0.6×10⁶ cells/mL. Equal volumes of CAR-T cells and tumor cells were mixed thoroughly. After 6 days of culture, CFSE fluorescence intensity was measured using flow cytometry. CAR-T cells without tumor cells served as a control group.

As depicted in FIG. 17, when analyzing the proliferation rate of CAR-T cells, all three types of bispecific CAR-T cells exhibited stimulation and expansion after co-culture with target cells. CFSE membrane-bound dyes were employed. As cells divided and proliferated, fluorescent cytoplasmic proteins were evenly distributed among second-generation cells, resulting in a reduction in fluorescence intensity half compared to first-generation cells. Similarly, the fluorescence intensity of divided third-generation cells was weaker than that of second-generation cells. This phenomenon was detectable and analyzable using flow cytometry under 488 nm excitation light. The continuous decrease in cell fluorescence intensity indicated cell division and proliferation. The value displayed represented the percentage of undivided cells, with lower values, indicating stronger expansion ability. Therefore, CD19/CD22 BS Loop CAR-T cells exhibited the highest expansion ability.

### Example 11

### Analysis of proliferation quantity by CD19/CD22 dual-target CAR-T cells with different structures

Tumor cells: Nalm6-GL, Nalm6-GL-KO19, Nalm6-GL-KO22, and K562.

CAR-T cells: Three types of CAR-T cells described in Example 2 were used: CD19/CD22 BS Loop CAR-T (amino acid sequence set forth in SEQ ID NO: 3), CD19/CD22 G4S Tan CAR-T (amino acid sequence set forth in SEQ ID NO: 1), and the CD19/CD22 LHL Tan CAR-T (amino acid sequence set forth in SEQ ID NO: 2), were starved to remove IL-2 24 hours in advance.

The CAR-T cells were co-cultured with tumor cells at an effector-target ratio (E: T) of 1:1.

The CAR-T cells were labeled using the PKH26 fluorescent labeling kit and adjusted to a density of 1×10⁶ cells/mL. Nalm6-GL, Nalm6-GL-KO19, and Nalm6-GL-KO22 tumor cells exhibited GFP fluorescence, while K562 cells were labeled with CFSE fluorescence following the steps outlined in Example 10. All four types of tumor cells were counted, diluted to 1×10⁶ cells/mL, and equal volumes of CAR-T cells and tumor cells were thoroughly mixed. After 6 days of culture, flow cytometry was conducted, and CAR-T cells were identified as GFP-negative or CFSE-negative and PKH26-positive. The CAR-T cells were counted to generate a CAR-T cell expansion chart.

As shown in FIG. 18, when analyzing the proliferation of CAR-T cells, all three types of bispecific CAR-T cells demonstrated stimulation and expansion following co-culture with target cells. In addition, the number of CD19/CD22 BS Loop CAR-T cells increased significantly more after co-culture with target cells, indicating that CD19/CD22 BS Loop CAR-T exhibited the highest proliferation capacity.

### Example 12

### Analysis of in vivo anti-tumor activity by CD19/CD22 bispecific CAR-T cells

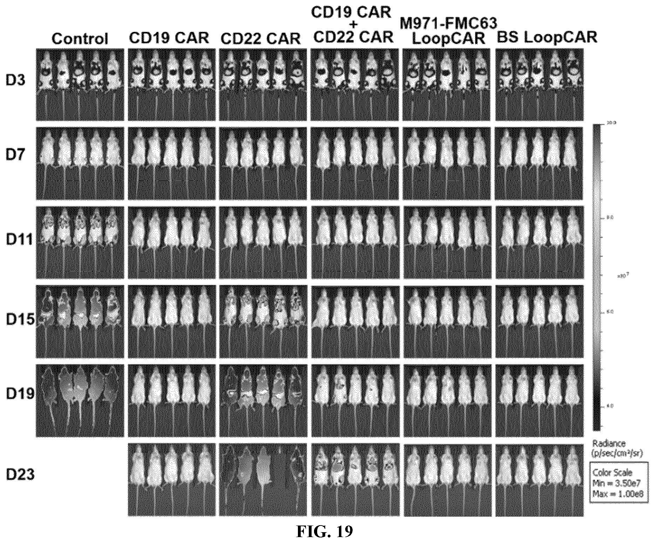

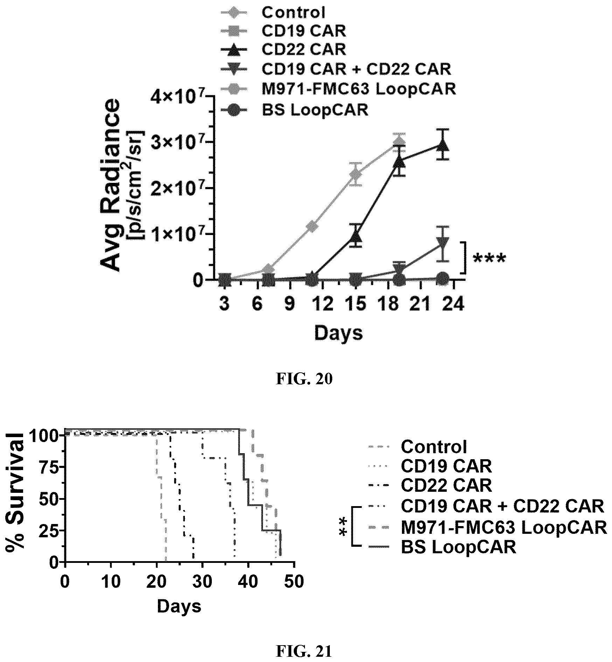

NCG mice (6-8 weeks old) were housed and treated under specific pathogen-free conditions. To evaluate the anti-tumor efficacy *in vivo,* mice were injected with 1×10⁶ Nalm6 tumor cells (double positive for CD19 and CD22) tagged with luciferase via the tail vein. The tumor model was successfully established once tumors grew and dispersed well in the NCG mice. Subsequently, the tumor model mice were divided into six groups and intravenously administered with CD19 CAR-T, CD22 CAR-T, CD19 CAR-T + CD22 CAR-T (1:1), M971-FMC63 Loop CAR-T, and CD19/CD22 BS Loop CAR-T cells of the present disclosure (each CAR-T cell dosage was 10×10⁶/mouse); the untreated group served as the control. The distribution of tumor cells in NCG mice was regularly monitored using an IVIS fluorescence imaging system. As depicted in FIG. 19 and FIG. 20, it is evident that the CD19 CAR-T, CD19/CD22 BS Loop CAR-T and M971-FMC63 Loop CAR-T of the present disclosure all exhibited desirable anti-leukemia effects on the Nalm6 tumor model. However, the treatment of CD22 CAR-T or the combination of monospecific CAR-T (CD19 CAR-T + CD22 CAR-T) failed to control the disease burden. Notably, the CD19/CD22 BS Loop CAR-T of the present disclosure, being structurally optimized to simultaneously target CD19 and CD22, did not compromise the *in vivo* anti-leukemia efficacy and exhibited kinetics consistent with previously reported CD19 CAR-T and other CD19/CD22 bispecific CAR-T cells. On day 37, the survival rate of mice treated with the two loop-structured CAR-T cells was 100%, while the survival rate of mice treated with a combination of monospecific CAR-T cells was 0% (P<0.0001, as shown in FIG. 21). In summary, it was demonstrated that the CD19/CD22 BS Loop CAR-T and M971-FMC63 Loop CAR-T of the present disclosure exhibited *in vivo* anti-tumor activity against CD19-positive and CD22-positive leukemias comparable to that of CD19 CAR-T cells.

### Example 13

### Analysis of in vivo anti-dual tumor immune escape activity by CD19/CD22 bispecific CAR-T cells

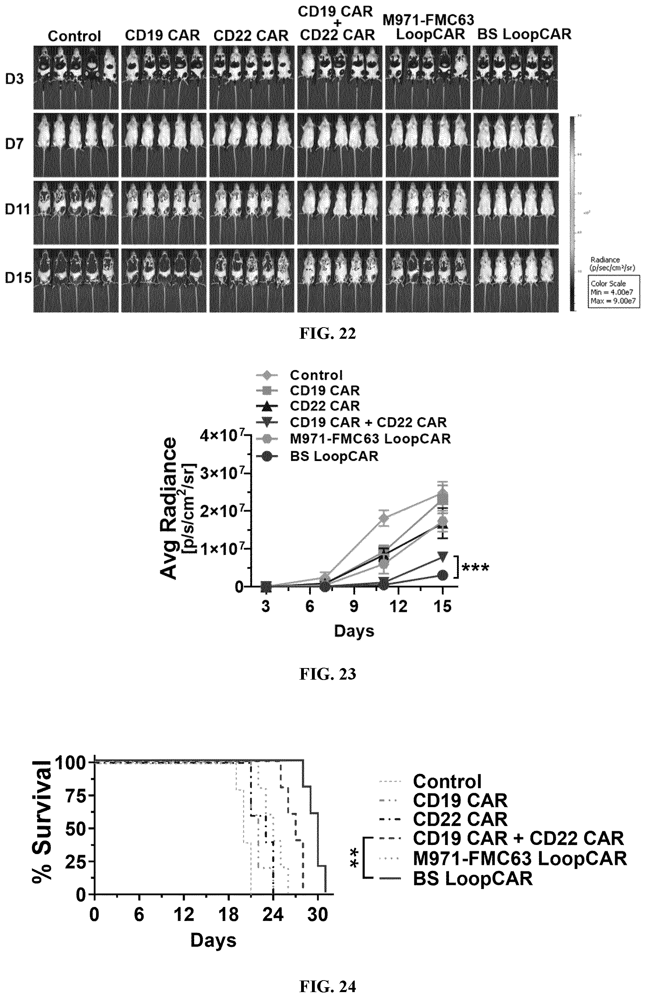

Mice were injected with a mixture of tumor cells consisting of Nalm6-GL-KO19 and Nalm6-GL-KO22 with luciferase through the tail vein. Once the tumors grew and dispersed well in NCG mice, a tumor model was successfully established. Subsequently, the tumor model mice were divided into six groups and intravenously administered with CD19 CAR-T, CD22 CAR-T, CD19 CAR-T + CD22 CAR-T, M971-FMC63 Loop CAR-T, and CD19/CD22 BS Loop CAR-T cells of the present disclosure (each CAR-T cell dosage was 10×10⁶/mouse); and the untreated group served as the control. The distribution of tumor cells in NCG mice was regularly monitored using the IVIS fluorescence imaging system, and the results were detailed in FIG. 22 and FIG. 23. It was observed that the CD19/CD22 BS Loop CAR-T of the present disclosure significantly inhibited the growth of heterogeneous tumors and achieved sustained disease remission; whereas CD19 CAR-T or CD22 CAR-T treatment failed to control tumor burden. Notably, compared with the CD19/CD22 BS Loop CAR-T of the present disclosure, treatment of M971-FMC63 Loop CAR-T and the combination of monospecific CAR-T cells had a poor tumor inhibition effect, suggesting the importance of selecting and optimizing the structure of the antibody domain and C-peptide of the CD19/CD22 BS Loop CAR-T of the present disclosure. Among all treatment groups, treatment with CD19/CD22 BS Loop CAR-T of the present disclosure increased the survival time of mice (FIG. 24). Correspondingly, the survival rate of mice treated with CD19/CD22 BS Loop CAR-T of the present disclosure on day 29 was 80%, whereas that of mice treated with the combination of monospecific CAR-T cells was 0% (P=0.0082). In summary, it was demonstrated that the CD19/CD22 BS Loop CAR-T of the present disclosure showed a significant and effective inhibitory effect against tumor antigen escape and exhibited a more sustained and effective tumor inhibitory effect than that of the traditionally reported M971-FMC63 Loop CAR-T cells. It further proves that bispecific CAR-T cells designed using the BS Loop CAR structure of the present disclosure could more effectively identify single-target tumor cells, thus effectively overcoming tumor recurrence caused by single-target antigen escape or tumor antigen heterogeneity, and achieving effective protection for tumor patients.

### Example 14

### Analysis of in vivo anti-triple tumor immune escape activity by CD19/CD22 bispecific CAR-T cells

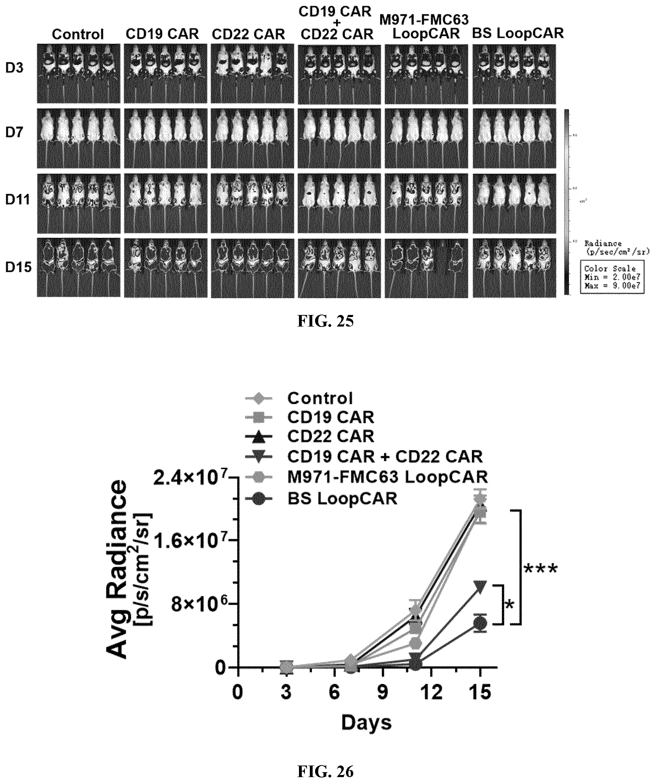

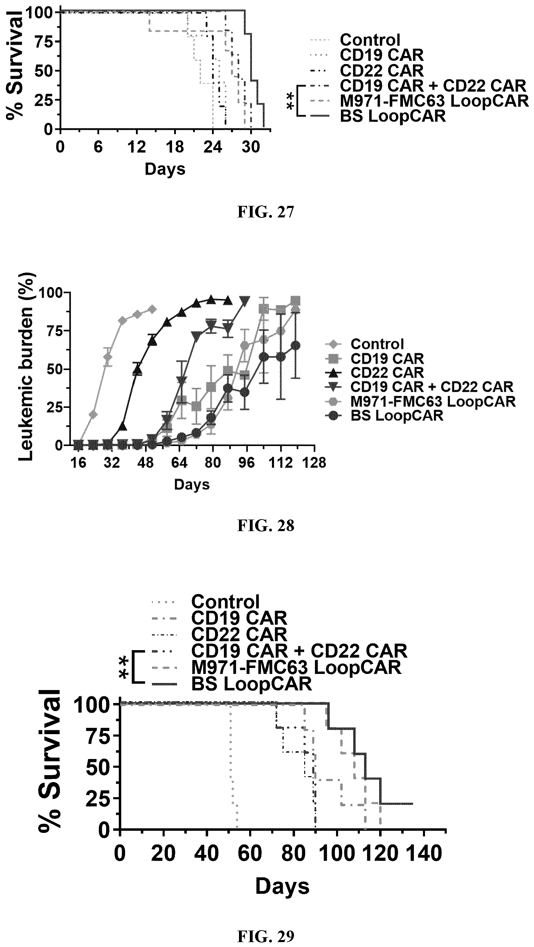

In the present disclosure, the efficacy of CD19/CD22 BS Loop CAR-T was evaluated in another immune escape model. In this model, mice were injected with a mixture of 1×10⁶ tumor cells (Nalm6: Nalm6-KO19: Nalm6-KO22 at a ratio of 1:1:1) via the tail vein. This tumor mixture represented an antigen-heterogeneous tumor, wherein some cells had lost antigen expression, thereby hindering the activation of corresponding CAR-T cells and anti-tumor immunity. Once tumors satisfactorily grew and dispersed in NCG mice, the tumor model was successfully established. The tumor model mice were divided into six groups and intravenously administered with CD19 CAR-T, CD22 CAR-T, CD19 CAR-T + CD22 CAR-T, M971-FMC63 Loop CAR-T, and CD19/CD22 BS Loop CAR-T cells of the present disclosure (each CAR-T cell dosage was: 10×10⁶/mouse); and untreated group served as control. The distribution of tumor cells in NCG mice was regularly monitored using the IVIS fluorescence imaging system. As depicted in FIG. 25 and FIG. 26, the treatment of CD19/CD22 BS Loop CAR-T and the combination of monospecific CAR-T of the present disclosure restrained the growth of heterogeneous tumors by the 15th day. However, CD19/CD22 BS Loop CAR-T exhibited significantly prolonged and sustained inhibitory effects on heterogeneous tumors. Conversely, both monospecific CAR-T and M971-FMC63 Loop CAR-T therapy failed to effectively control tumor burden. Furthermore, the survival period of mice treated with CD19/CD22 BS Loop CAR was significantly extended (*P*<0.05, as shown in FIG. 27). In summary, it was demonstrated that CD19/CD22 BS Loop CAR of the present disclosure elicited the most potent anti-tumor response against wild-type leukemia or heterogeneous leukemia with antigen loss or downregulation under stress response, potentially preventing immune escape and tumor recurrence induced by monospecific CAR-T cell therapy. CD19/CD22 BS Loop CAR-T exhibited superior efficacy compared to monospecific CAR-T combination therapy, underscoring the strategic advantages of structurally optimized bispecific CD19/CD22 CAR cells.

### Example 15

### Analysis of anti-tumor activity by CD19/CD22 bispecific CAR-T cells on early PDX model

In this study, the *in vivo* efficacy of the CD19/CD22 BS Loop CAR-T of the present disclosure was further investigated on a clinically relevant early PDX model of B-ALL. Mice were intravenously injected with 5×10⁶ primary B-ALL patient tumor cells via the tail vein. After 7 days, the early PDX model was established successfully once tumors had sufficiently grown and dispersed in the NCG mice. The tumor-bearing mice were divided into six groups and intravenously administered with CD19 CAR-T, CD22 CAR-T, CD19 CAR-T + CD22 CAR-T, M971-FMC63 Loop CAR-T, and CD19/CD22 BS Loop CAR-T cells of the present disclosure separately on day 7 (each CAR-T cell dosage was 30×10⁶/mouse); the untreated group served as the control. Subsequently, the leukemia burden in peripheral blood was measured weekly. In the early PDX model, leukemia cells were effectively eradicated following CAR-T cell infusion (FIG. 28); however, mice treated with CD22 CAR-T failed to control the disease, leading to relapse from the 4th week onwards. Furthermore, mice treated with CD19 CAR-T and the combination of monospecific CAR-T exhibited increasingly severe relapses by day 64. Conversely, both CD19/CD22 BS Loop CAR-T and M971-FMC63 Loop CAR-T cells of the present disclosure demonstrated prolonged anti-tumor effects. In the later stage of PDX model testing, only mice treated with CD19/CD22 BS Loop CAR-T exhibited better control of tumor burden to some extent, resulting in lower tumor burden in the mice. CD19/CD22 BS Loop CAR-T maintained mice in a long-term state of tumor remission and extended the survival curve of mice (FIG. 29). Therefore, the above findings confirmed the safety and effective anti-tumor efficacy of the CD19/CD22 BS Loop CAR of the present disclosure.

### Example 16

### Analysis of anti-tumor activity by CD19/CD22 bispecific CAR-T cells on late PDX model

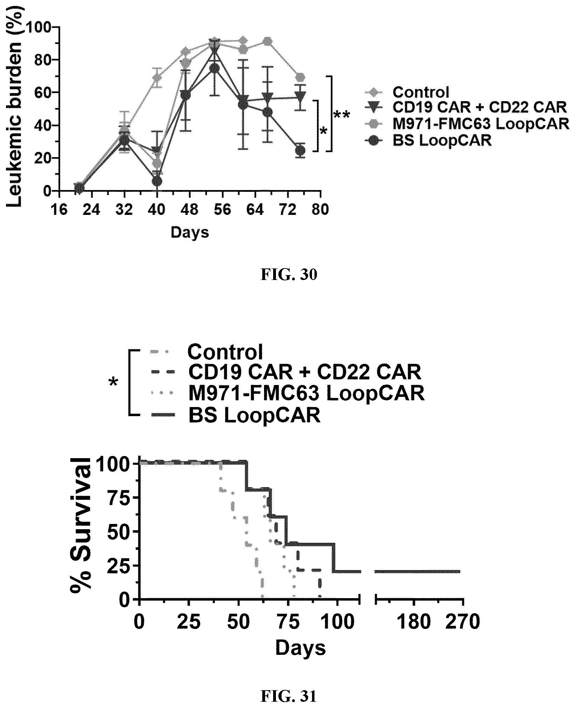

Mice were injected with 5×10⁶ primary B-ALL patient tumor cells via the tail vein. After 32 days, the late PDX model was successfully established once tumors had sufficiently grown and dispersed in the NCG mice. The tumor-bearing mice were divided into six groups and intravenously administered with CD19 CAR-T, CD22 CAR-T, CD19 CAR-T + CD22 CAR-T, M971-FMC63 Loop CAR-T, and CD19/CD22 BS Loop CAR-T cells of the present disclosure separately on day 32 (each CAR-T cell dosage was 30×10⁶/mouse); the untreated group served as the control. Subsequently, the leukemia burden and T cell persistence in peripheral blood were measured weekly. As depicted in FIG. 30, in the late PDX model, mice in the control group exhibited rapid disease progression, with the proportion of leukemia cells in peripheral blood exceeded 80% after 7 weeks. In contrast, all CAR-T treatment groups effectively controlled the disease by eliminating leukemia cells in the early stages of post-infusion. However, as the disease progressed, mice treated with M971-FMC63 Loop CAR-T or the combination of monospecific CAR-T cells failed to eradicate primary B-ALL cells, whereas the CD19/CD22 BS Loop CAR-T treatment group of the present disclosure demonstrated sustained and long-lasting anti-leukemia activity. Additionally, the survival rate of mice treated with the CD19/CD22 BS Loop CAR-T was significantly prolonged (as shown in FIG. 31). These results unequivocally demonstrate that the CD19/CD22 BS Loop CAR of the present disclosure, which targets CD19 and CD22, exhibited promising therapeutic efficacy in early or late B-ALL PDX models, positioning it as a compelling candidate for clinical transformation.

### Example 17

### Design and construction of Her2/IGF1R BS Loop CAR

### Preparation of Her2/IGF1R BS Loop CAR (SEQ ID NO: 12)

The 4D5 scFv V_{L} fragment was amplified using the forward primer sequence GATATCCAGATGACCCAGTCCCCG and the reverse primer sequence TTTTTTAGTCTCCTCAGTACGTTTGATCTC. Similarly, the natural ligand Adnectin fragment was amplified using the forward primer sequence GAGATCAAACGTACTGAGGAGACTAAAAAATATCAGTCTGACATCGTGAGCGAC and the reverse primer sequence CACCAGCTGAACCTCTTTTTCGTAATTATAAGTGTAAGATTGGCTAGGCTTGTC. Additionally, the 4D5 scFv V_{H} fragment was amplified using the forward primer sequence AATTACGAAAAAGAGGTTCAGCTGGTG and the reverse primer sequence atccagaggttgattgtcgacttagcgagggggcagggc. The above three PCR products were recombined into one target gene fragment after overlapping PCR amplification. The Her2/IGF1R BS Loop CAR sequence was included in the above primer sequence (in uppercase). The PCR system and reaction conditions were detailed in Table 1 and Table 2 in Example 1, respectively.

The procedures for PCR product recovery, vector digestion, and homologous recombination, as well as plasmid transformation and maxiprep were identical to those described in Example 1.

### Example 18

### Preparation of Her2 and IGF1R targeting CAR-T cells

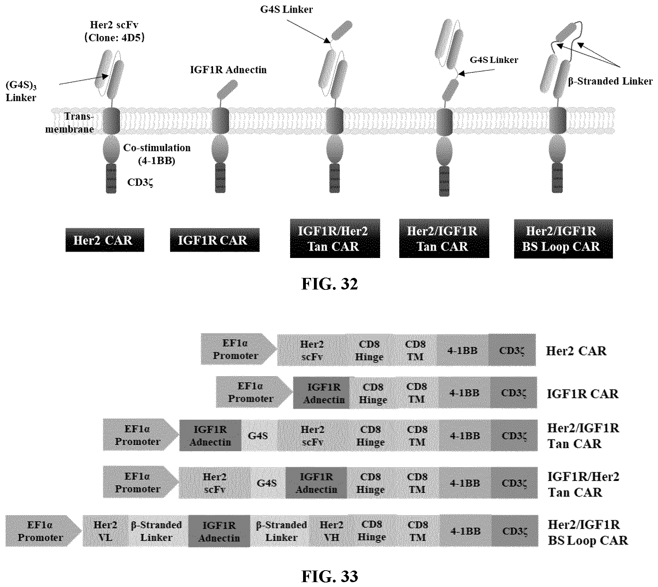

The plasmids containing sequences for Her2 CAR (amino acid sequence in SEQ ID NO: 13), IGF1R CAR (as defined in SEQ ID NO: 9), IGF1R/Her2 Tan CAR (as defined in SEQ ID NO: 10), Her2/IGF1R Tan CAR (as defined in SEQ ID NO: 11), or Her2/IGF1R BS Loop CAR (as defined in SEQ ID NO: 12) were utilized for lentivirus preparation. The structural schematic diagram and the molecular structures of the aforementioned five CAR were detailed in FIG. 32 and FIG. 33, respectively. The subsequent procedures for PBMC extraction, recovery, activation, lentivirus preparation, viral transduction, and CAR-T cell preparation followed the protocol outlined in Example 1.

### Example 19

### Analysis of tumor cell killing by Her2 and IGF1R-targeted CAR-T cells

Tumor cells: SKBR3 (Her2+++/IGF1R+), MDA-MB-453 (Her2++/IGF1R+), and MDA-MB-231 (Her2+/IGF1R+).

CAR-T cells: The five types of CAR-T cells described in Example 18, as well as the mixed CAR-T cells comprising Her2 CAR-T and IGF1R CAR-T, were starved to remove IL-2 for 24 hours in advance.

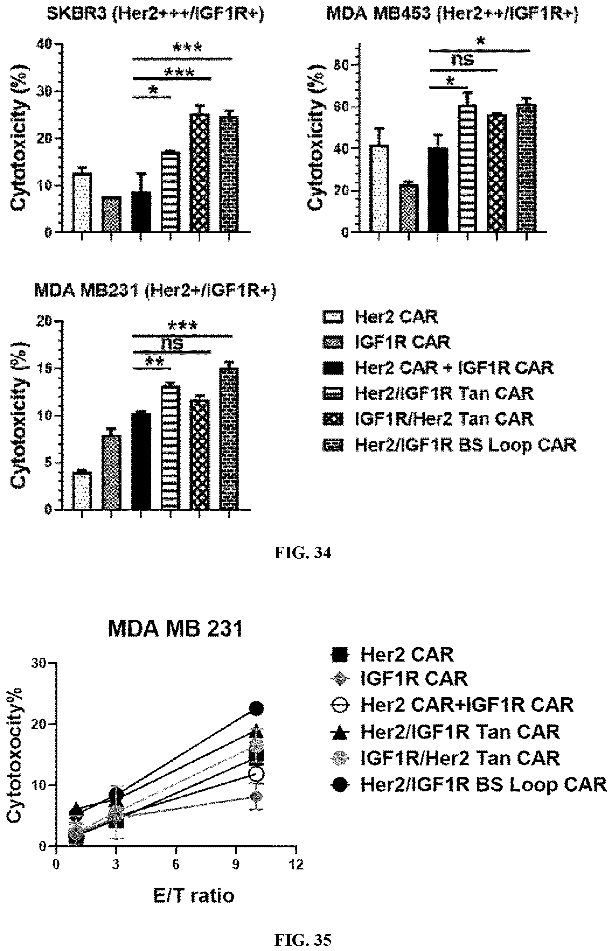

The CAR-T cells were co-cultured with tumor cells at an effector-target ratio (E: T) of 10:1.
1) Cell counting: The specified tumor cells and CAR-T cells were resuspended into RPMI-1640 medium for cell counting. If the tumor cells were challenging to resuspend and tended to clump, they were allowed to settle for a period, then the supernatant was collected and mixed for cell counting and subsequent experiments.
2) Cell dilution: The CAR-T cells were diluted to 2×10⁶ cells/mL. The tumor cells were counted and adjusted to a density of 0.2×10⁶ cells/mL. Equal volumes of the density-adjusted CAR-T cells and tumor cells were mixed and added at 100 µL to each well into a 96-well plate. The group mixed with 50 µL of tumor cells and 50 µL of medium in equal volumes served as the positive control group.
3) Detection of LDH: After 24 hours, 10 µL/well lysis buffer (10×) was added to the positive control group, and the cells were fully lysed at 37 °C for 30 minutes. After centrifugation, 30 µL/well of the supernatant was transferred to a new 96-well flat-bottomed plate and added with 30 µL/well of chromogenic solution (using a Promega Cytotoxicity Detection Kit) accordingly. The plate was then incubated at room temperature in the dark for 20 minutes, and 30 µL/well of stop solution was added, followed by measurement of the absorbance value at 490 nm.
4) OD₄₉₀ analysis: The OD₄₉₀ values of the positive control and the negative control group were separately averaged to calculate the killing percentage efficiency using the formula: % Lysis = (OD₄₉₀ value corresponding to each well - average of negative control group)/ (average of positive control group - average of negative control group) ×100. As illustrated in FIG. 34, compared with Her2 CAR-T, IGF1R CAR-T and mixed CAR-T cells, all three bispecific CAR-T cells exhibited enhancing killing activity against tumor cells with varying Her2 expression levels. However, notably, the Her2/IGF1R BS Loop CAR-T cells of the present disclosure demonstrated the most significant killing activity compared with the other candidates. These results indicate that the CAR-T cells utilizing the BS Loop structure of the present disclosure possess optimal and desirable consistency in the activity of dual-target tumor recognition.

### Example 20

### Analysis of tumor cell killing by Her2 and IGF1R-targeted CAR-T cells at different effector-target ratios

Tumor cells: MDA-MB-231 (Her2+/IGF1R+).

CAR-T cells: The five types of CAR-T cells described in Example 18, as well as the mixed CAR-T cells comprising Her2 CAR-T and IGF1R CAR-T, were starved to remove IL-2 for 24 hours in advance.

The CAR-T cells were co-cultured with tumor cells at an effector-target ratio (E: T) of 10:1, 3:1, and 1:1.
1) Cell counting: The specified tumor cells and CAR-T cells were resuspended into RPMI-1640 medium for cell counting. If the tumor cells were difficult to resuspend and tend to clump, they were allowed to settle for a period of time, after which the supernatant was collected and mixed for cell counting and subsequent experiments.
2) Cell dilution: The CAR-T cells were diluted to 2×10⁶ cells/mL, 0.6×10⁶ cells/mL, and 0.2×10⁶ cells/mL. The tumor cells were counted and adjusted to a density of 0.2×10⁶ cells/mL. Equal volumes of the density-adjusted CAR-T cells and tumor cells were mixed and added at 100 µL to each well into a 96-well plate. The group mixed with 50 µL of tumor cells and 50 µL of medium in equal volumes served as the positive control group, while the group mixed with 50 µL of tumor cells and 50 µL of irrelevant target CAR-T cells in equal volumes served as the negative control group. Additionally, the group mixed with 50 µL of tumor cells and 50 µL of medium in equal volumes without adding the lysis buffer served as the complete-negative control group.
3) Detection of LDH: After 24 hours, 10 µL/well lysis buffer (10×) was added to the positive control group, and the cells were fully lysed at 37 °C for 30 min. After centrifugation, 30 µL/well of the supernatant was transferred to a new 96-well flat-bottomed plate and added with 30 µL/well of chromogenic solution (using a Promega Cytotoxicity Detection Kit) accordingly. The plate was then incubated at room temperature in the dark for 20 minutes, followed by the addition of 30 µL/well stop solution, and measurement of the absorbance value at 490 nm.
4) OD₄₉₀ analysis: The OD₄₉₀ values of the positive control and the negative control group were separately averaged to calculate the killing percentage efficiency using the formula: % Lysis = (OD₄₉₀ value corresponding to each well - average of negative control group)/ (average of positive control group - average of all-negative control group) ×100. The results were detailed in FIG. 35. Compared with Her2 CAR-T, IGF1R CAR-T and mixed CAR-T cells, all three bispecific CAR-T cells exhibited enhanced killing activity against MDA-MB-231 tumor cells. However, notably, the Her2/IGF1R BS Loop CAR-T cells of the present disclosure showed better killing activity compared with the other candidates. These findings suggest that the CAR-T cells with the BS Loop structure of the present disclosure possess desirable and optimal consistency in the activity of dual-target tumor recognition.

### Example 21

### Analysis of cytokine release from Her2 and IGF1R-targeted CAR-T cells

Tumor cells: MDA-MB-231 (Her2+/IGF1R+)

CAR-T cells: The five types of CAR-T cells described in Example 18, as well as the mixed CAR-T cells comprising Her2 CAR-T and IGF1R CAR-T, were starved to remove IL-2 for 24 hours in advance.

The CAR-T cells were co-cultured with tumor cells at an effector-target ratio (E: T) of 10:1.

After centrifuging the CAR-T cells to remove the supernatant, they were resuspended, counted, and diluted to a concentration of 2×10⁶ cells/mL; the tumor cells were centrifuged to remove the supernatant, then resuspended, counted, and diluted to a concentration of 0.2×10⁶ cells/mL. Equal volumes of CAR-T cells and tumor cells were mixed. After 24 hours of co-culture, the supernatant was collected and frozen at -20 °C for subsequent detection of cytokines. The method for cytokine detection by ELISA was that described in Example 6.

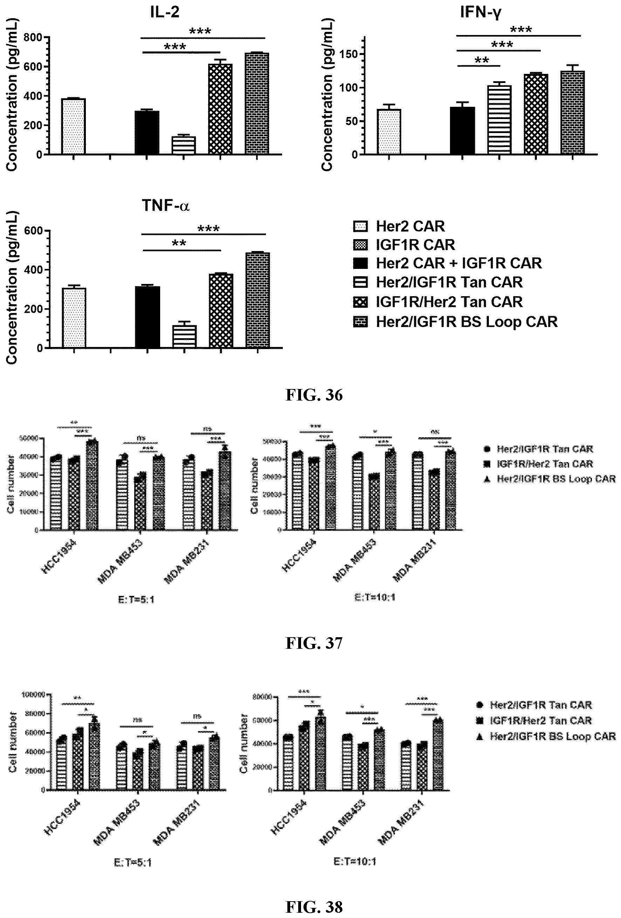

As shown in FIG. 36, compared to Her2 CAR-T, IGF1R CAR-T and mixed CAR-T cells, the three bispecific CAR-T cells all exhibited significantly enhanced release of IFN-γ cytokine against Her2+/IGF1R+MDA-MB-231 tumor cells. However, notably, the Her2/IGF1R BS Loop CAR-T cells of the present disclosure consistently and significantly released IL-2, IFN-γ and TNF-α cytokines compared with the other candidates. These results demonstrate that the CAR-T cells with the BS Loop structure of the present disclosure exhibit desirable consistency in activating dual-target tumor recognition.

### Example 22

### Analysis of the expansion ability of Her2/IGF1R dual-target CAR-T cells with different structures

Tumor cells: HCC1954 (Her2+++/IGF1R+), MDA-MB-453 (Her2++/IGF1R+), and MDA-MB-231 (Her2+/IGF1R+).

CAR-T cells: IGF1R/Her2 Tan CAR-T (amino acid sequence set forth in SEQ ID NO: 10) cells, Her2/IGF1R Tan CAR-T (amino acid sequence set forth in SEQ ID NO: 11) cells, and Her2/IGF1R BS Loop CAR-T (amino acid sequence set forth in SEQ ID NO: 12) cells as described in Example 18.

The CAR-T cells were co-cultured with tumor cells at an effector-target ratio (E: T) of 5:1 and 10:1.

The CAR-T cells were labeled using the CFSE fluorescent labeling kit according to the manufacturer's instructions and adjusted to a density of 2×10⁶ cells/mL. Subsequently, the tumor cells were counted and adjusted to densities of 0.4×10⁶ cells/mL and 0.2×10⁶ cells/mL, respectively. Equal volumes of the CAR-T cells and tumor cells were thoroughly mixed. The cells were cultured for 6 and 10 days. In the 10-day group, the medium was changed, and IL-2 was added on day six and a half to reach a final concentration of 300 IU/mL. Flow cytometry was conducted, and CFSE-positive cells represented the CAR-T cells. The number of CAR-T cells was counted to generate a CAR-T cell expansion chart.

The expansion results are depicted in FIG. 37 and FIG. 38 After co-culture three types of bispecific CAR-T cells with target cells expressing different antigen levels for 6 and 10 days, all the CAR-T cells showed broad-spectrum and effective stimulation and expansion. Notably, the number of Her2/IGF1R BS Loop CAR-T cells increased significantly, demonstrating the highest expansion capacity among all the CAR-T candidates.

### Example 23

### Analysis of the proliferation ability of Her2/IGF1R dual-target CAR-T cells with different structures

Tumor cells: MDA-MB-231 (Her2+/IGF1R+).

CAR-T cells: IGF1R/Her2 Tan CAR-T (amino acid sequence set forth in SEQ ID NO: 10) cells, Her2/IGF1R Tan CAR-T (amino acid sequence set forth in SEQ ID NO: 11) cells, and Her2/IGF1R BS Loop CAR-T (amino acid sequence set forth in SEQ ID NO: 12) cells as described in Example 18.

The CAR-T cells were co-cultured with tumor cells at an effector-target ratio (E: T) of 5:1 and 10:1.

The CAR-T cells were labeled using the CFSE fluorescent labeling kit according to the manufacturer's instructions and adjusted to a density of 2×10⁶ cells/mL. Subsequently, the tumor cells were counted and adjusted to densities of 0.4×10⁶ cells/mL and 0.2×10⁶ cells/mL, respectively. Equal volumes of CAR-T cells and tumor cells were thoroughly mixed. The cells were cultured for 6 and 10 days. In the 10-day group, the medium was changed, and IL-2 was added on day six and a half to reach a final concentration of 300 IU/mL; and the cells were used to measure the intensity of CFSE fluorescence by flow cytometry. CAR-T cells without the addition of tumor cells served as a control group.

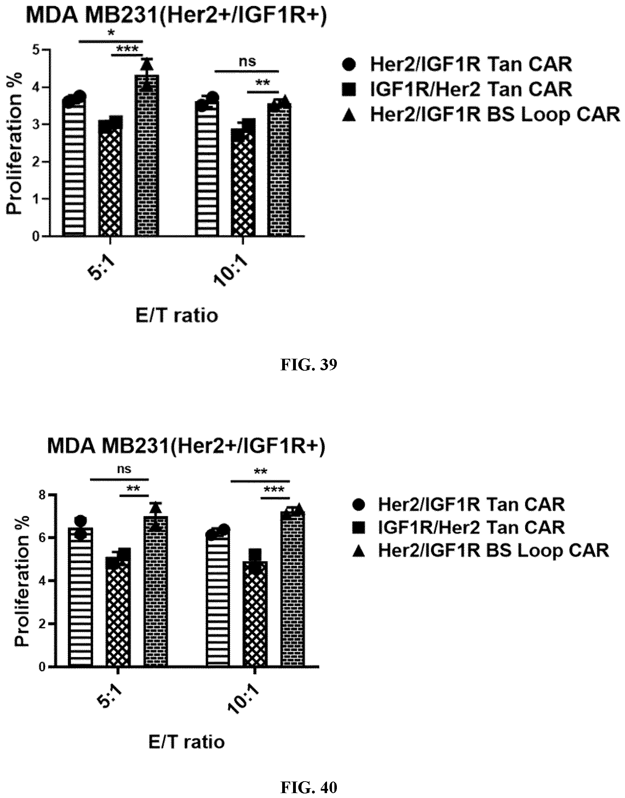

The proliferation results were depicted in FIG. 39 and FIG. 40. After co-culture three types of bispecific CAR-T cells with target cells expressing different antigen levels for 6 and 10 days, all the CAR-T cells exhibited broad-spectrum and effective stimulation and expansion. CFSE membrane-bound dyes were employed. As cells divided and proliferated, fluorescent cytoplasmic proteins were evenly distributed among second-generation cells, resulting in their fluorescence intensity being halved compared to first-generation cells. By analogy, the fluorescence intensity of the divided third-generation cells was weaker than that of the second-generation cells. This phenomenon could be detected and analyzed using flow cytometry under 488 nm excitation light. By continuously detecting the decrease in cell fluorescence intensity, the migration percentage was further analyzed to reveal the status of cell division and proliferation. The value displayed represented the percentage of divided cells, indicating that the higher the value, the stronger the proliferation ability. Therefore, Her2/IGF1R BS Loop CAR-T cells exhibited the highest proliferation capability among all the CAR-T candidates.

### Example 24

### Analysis of immunosuppressive receptor expression in CAR-T cells targeting Her2 and IGF1R

Tumor cells: HCC1954 (Her2+++/IGF1R+), MDA-MB-453 (Her2++/IGF1R+), and MDA-MB-231 (Her2+/IGF1R+).

CAR-T cells: The five types of CAR-T cells described in Example 18, as well as the mixed CAR-T cells comprising Her2 CAR-T and IGF1R CAR-T.

The effector-target ratio (E: T) of CAR-T cells to tumor cells was set at 3:1 and 10:1.

After centrifuging and resuspending the CAR-T cells, they were diluted to a concentration of 2× 10⁶ cells/mL. The tumor cells were centrifuged and resuspended, then diluted to 0.6×10⁶ cells/mL and 0.2×10⁶ cells/mL. Equal volumes of CAR-T cells and tumor cells were mixed, and co-cultured for 5 days. Subsequently, the cells were used to detect the expression of inhibitory molecules. Flow cytometry antibodies LAG3-FITC, TIM3-APC and PD-1-PE were stained on ice for 1 hour, followed by washing twice with FACS, and then resuspension for flow cytometry analysis.

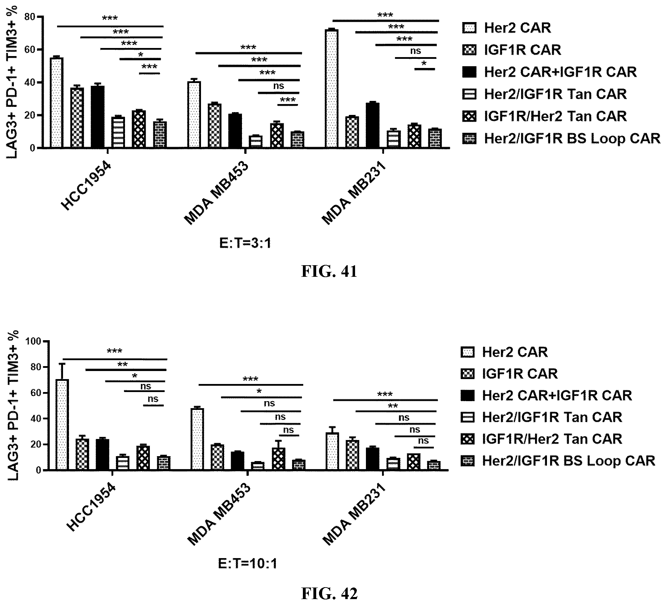

As shown in FIG. 41 and FIG. 42, when analyzing the total proportions of LAG3+, TIM3+, and PD-1+ CAR-T cell, it is evident that the three bispecific CAR-T cells showed lower expression of inhibitory receptors after co-culture with the target cells, compared to Her2 CAR-T, IGFIR CAR-T and the mixed CAR-T cells. Among them, the total percentage of LAG3+, TIM3+, and PD-1+ expression was lowest in Her2/IGF1R BS Loop CAR-T cells, indicating reduced expression of broad-spectrum inhibitory molecules against target cell lines with different Her2 antigen expression levels. Therefore, Her2/IGF1R BS Loop CAR-T cells, with the lowest expression of inhibitory receptors, are advantageous for further functional activity.

The foregoing descriptions represent only preferred implementations of the present disclosure. It should be noted that for a person of ordinary skill in the art, several improvements and modifications may be made without departing from the principles of the present disclosure. These improvements and modifications should also be considered falling within the scope of protection of the present disclosure.

## Claims

1. A chimeric antigen receptor (CAR), wherein the CAR comprises: an antigen-binding domain, a connecting peptide (C-peptide), a hinge region, a transmembrane domain, a 4-1BB co-stimulatory signaling domain, and a CD3ζ signaling domain; and wherein the antigen-binding domain comprises a dual-target recognition molecule.

2. The CAR according to claim 1, wherein the dual-target recognition molecule comprises a fibronectin, an affibody, a lipocalin, a bicyclic peptide, an ankyrin repeat protein, a Fyn kinase derivative, a Kunitz domain, an E7 immune protein, a lymphocyte receptor variable region, a single domain antibody, a complete antibody, an antibody fragment, and an aptamer.

3. The CAR according to claim 2, wherein the antigen or receptor recognized by the dual-target recognition molecule is selected from CD19, CD20, CD22, CD33, BCMA, CD123, CD133, CD38, CD39, CD138, CD73, CD30, CD7, CD33, CS1, CD56, CD4, CLL1, Lewis Y, CD138, ROR1, Her2/neu, IGF1R, EGFR, Her3, Her4, VEGFR-1, VEGFR-2, VEGFR-3, mesothelin, GPC2, GPC3, CD33/IL3Ra, c-Met, MUC-1, PSMA, CAIX, CEA, PSCA, GD2, glycolipid F77, EGFRvIII, EpCAM, CD70, Claudin 18.2, IL-13, CD133, CD171, FAP, FBP, PD-L1, NYESO-1, and MAGEA3.

4. The CAR according to claim 1, wherein the C-peptide comprises a G4S Linker, a Long Hinge Lama Linker, a β-Stranded Loop Linker; and wherein the forward and reverse amino acid sequences of the β-Stranded Loop Linker comprise EETKKYQS and SYTYNYEK.

5. The CAR according to claim 3, wherein the antigen-binding domain comprises an anti-CD19 scFv and an anti-CD22 nanobody, or comprises an anti-Her2 scFv and a ligand capable of recognizing IGF1R.

6. The CAR according to claim 5, wherein the antigen-binding domain comprises an anti-CD22 nanobody and an anti-CD19 scFv sequentially ligated in series with the C-peptide, or comprises a variable region of light chain (VL) of the anti-CD19 scFv, an anti-CD22 nanobody, and a variable region of heavy chain (VH) of the anti-CD19 scFv sequentially ligated in series with the C-peptide, or comprises the VH of the anti-CD19 scFv, the anti-CD22 nanobody, and the VL of the anti-CD19 scFv sequentially ligated in series with the C-peptide.

7. The CAR according to claim 6, wherein the anti-CD19 scFv comprises the anti-CD19 monoclonal antibody FMC63, and the anti-CD22 nanobody comprises anti-CD22 monoclonal antibodies CD22-Nb25, CD22-Nb35, and CD22-Nb45.

8. The CAR according to claim 5, wherein the antigen-binding domain comprises a ligand capable of recognizing IGF1R and the anti-Her2 scFv sequentially ligated in series with the C-peptide, or comprises the anti-Her2 scFv and the ligand capable of recognizing IGFIR sequentially ligated in series with the C-peptide, or comprises a VL of the anti-Her2 scFv, the ligand capable of recognizing IGF1R, and a VH of the anti-Her2 scFv sequentially ligated in series with the C-peptide, or comprises the VH of the anti-Her2 scFv, the ligand capable of recognizing IGF1R, and the VL of the anti-Her2 scFv sequentially ligated in series with the C-peptide.

9. The CAR according to claim 8, wherein the anti-Her2 scFv comprises an anti-Her2 monoclonal antibody 4D5, and the ligand capable of recognizing IGF1R is Adnectin.

10. The CAR according to any one of claims 1 to 9, wherein the CAR comprises amino acid sequences as set forth in SEQ ID NO: 1 to SEQ ID NO: 5 and SEQ ID NO: 10 to SEQ ID NO: 12.

11. A recombinant lentivirus, wherein the recombinant lentivirus is obtained by co-transfection of a viral vector containing the CAR according to any one of claims 1 to 10 with a mammalian cell.

12. A CAR-T cell, wherein the CAR-T cell expresses the CAR according to any one of claims 1 to 9; or, the amino acid sequences of the CAR according to claim 10 are integrated into a genome of the CAR-T cell; or, the CAR-T cell comprises the recombinant lentivirus according to claim 11.

13. Use of the CAR according to any one of claims 1 to 10, the recombinant lentivirus according to claim 11, or the CAR-T cell according to claim 12 in preparation of a drug.
